# EUROPEAN PATENT APPLICATION

(11) **EP 4 597 508 A1**
(43) Date of publication of application: **06.08.2025**
(21) Application number: 25150469.2
(22) Date of filing: 07.01.2025
(51) Int. Cl.: G16H 10/60, G16H 15/00, G16H 50/30, G16H 50/70

(54) **HEALTH EQUITY ASSESSMENT SYSTEM FOR CLINICAL DATA PRODUCTS**

(30) Priority: 30.01.2024 US 202418427441
(71) Applicant: GE Precision Healthcare LLC, Waukesha, WI 53188 (US)
(72) Inventor: VESTO, Guy R., Milwaukee, 53226 (US); MISHRA, Isani, 560066 Bengaluru (IN); ZHANG, Wei, Shanghai, 201203 (CN)
(74) Representative: Kilburn & Strode LLP

(57) **Abstract**

A confidence and health equity assessment system (102, 214) is provided for AI clinical data products (CDPs (1102, 206, 175, 402)) that may be launched via a clinical workflow application (160, 212). When a CDP (904, 1102, 206, 175, 302, 402) is launched, a rating tool micro-application of the confidence and health equity assessment system (102, 214) may be launched. When conditions are met, the rating tool micro-application may request clinical feedback from the user regarding the equitability, suitability, appropriateness, accuracy, or quality of an output of the CDP (904, 1102, 206, 175, 302, 402). The clinical feedback may include demographic data with respect to various disparity factors of a patient population analyzed by the CDP (904, 1102, 206, 175, 302, 402). After aggregation of clinical feedback, the confidence and health equity assessment system (102, 214) performs a health equity assessment (408) of the CDP (904, 1102, 206, 175, 302, 402) and generates a confidence rating of the CDP (904, 1102, 206, 175, 302, 402), where the confidence rating is a multi-dimensional score indicating a degree of confidence that the output of the CDP is not affected by algorithmic bias for various disparity factors.

## Description

### TECHNICAL FIELD

Embodiments of the subject matter disclosed herein relate generally to AI- based clinical tools, and more specifically, identifying bias in AI algorithms used in the tools.

### BACKGROUND

Clinical Decision Support (CDS) tools deployed by medical device/product manufacturers that rely on artificial intelligence (AI) models are backed by clinical studies for regulatory approval. However, these clinical studies may not have adequate coverage of a patient population with respect to the geography, race, age, gender, a variety of disease conditions, clinical study duration, etc. The lack of adequate coverage may lead to bias in the AI algorithms used by the CDS tools, where results generated by the CDS tools may be of higher quality for certain portions of the patient population, and of lower quality for other portions of the patient population. Systematic detection and monitoring of bias in AI algorithms at scale is a challenging problem for which few technical solutions exist. In some approaches, clinical experts can flag incorrect or incomplete AI insights, notifications, alerts, alarms, or recommendations. However, analyzing the health equity impact on a broad group of patient cohorts and labeling (re)training data for AI models can be both time consuming and daunting, especially for clinicians and nurses that are already overburdened.

### SUMMARY

In one example, the current disclosure addresses the issues described above with a health equity assessment system, comprising a processor and a non-transitory memory storing instructions that when executed, cause the processor to summarize a clinical context of an analysis performed by a clinical data product (CDP) on patient data; perform a health equity assessment of the CDP based on the summarized clinical context, using one or more disparity assessment models trained to assess an algorithmic bias of the CDP towards a specific patient population; calculate a confidence score for the CDP based on the health equity assessment, the confidence score indicating a degree of confidence that an analysis of a patient using the CDP does not suffer from algorithmic bias; and store the confidence score in a database.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a block schematic diagram of a health equity assessment system, in accordance with one or more embodiments of the present disclosure;
FIG. 2 shows a high-level block schematic diagram illustrating a workflow for generating an assessment of health equity of a CDP, in accordance with one or more embodiments of the present disclosure;
FIG. 3 shows a block schematic diagram illustrating a flow of data through various data ports of a CDP within the framework of the health equity assessment system, in accordance with one or more embodiments of the present disclosure;
FIG. 4 shows a block schematic diagram illustrating a CDP marketplace, in accordance with one or more embodiments of the present disclosure;
FIG. 5 is a flowchart showing an exemplary method for collecting a rating of a CDP selected by a clinician to be used by the health equity assessment system, in accordance with one or more embodiments of the present disclosure;
FIG. 6 is a flowchart showing an exemplary method for generating a health equity assessment of a CDP using the health equity assessment system, in accordance with one or more embodiments of the present disclosure;
FIG. 7 is a flowchart showing an exemplary method for displaying health equity assessment data associated with various CDPs, in accordance with one or more embodiments of the present disclosure;
FIG. 8 is a flowchart showing an exemplary method for determining drift in a model of a CDP, in accordance with one or more embodiments of the present disclosure;
FIG. 9 shows an exemplary CDP used to output an alert based on patient data, in accordance with one or more embodiments of the present disclosure;
FIG. 10 shows an exemplary set of control elements that may be displayed in a clinical workflow application to collect a rating of a CDP, in accordance with one or more embodiments of the present disclosure;
FIG. 11 is a first image of an exemplary graphical user interface (GUI) for a digital catalog of CDPs including health equity assessment data, in accordance with one or more embodiments of the present disclosure; and
FIG. 12 is a second image of the exemplary GUI for the digital catalog of CDPs including health equity assessment data, in accordance with one or more embodiments of the present disclosure.

The drawings illustrate specific aspects of the described systems and methods. Together with the following description, the drawings demonstrate and explain the structures, methods, and principles described herein. In the drawings, the size of components may be exaggerated or otherwise modified for clarity. Well-known structures, materials, or operations are not shown or described in detail to avoid obscuring aspects of the described components, systems and methods.

### DETAILED DESCRIPTION

Care providers within a healthcare system may use Clinical Data Products (CDPs) to support clinical decision-making, identify patterns in patient data or patient population data, provide treatment suggestions, and/or other clinical tasks. A CDP is a digital asset that is created by a company or organization that aggregates and/or analyzes data to provide insights and knowledge to users. The CDP is one of the foundational principles referred to "Data as a Product" from a Data Mesh architecture. There are many formats in which data can be offered as a product, including APIs, datasets, data feeds, visualizations, reports, dashboards, notebooks, insights delivered via email or messaging, AI-based tools, such as machine learning (ML) or deep learning (DL) models, rule-based algorithms, etc. The goal of a CDP is to provide value to users by enabling them to make data-driven decisions or solve specific problems.

Health equity is the state in which everyone has a fair and just opportunity to attain their highest level of health. While health equity initiatives seek to reduce health disparities and enable optimal health for everyone, many organizations struggle to measure health equity progress, bias, and fairness in how CDPs are used. The human cost of biased AI algorithms in healthcare can be significant, and may include delayed or inaccurate diagnosis and treatment. Biased AI algorithms can further exacerbate existing healthcare disparities, by disproportionately impacting certain groups of patients. This can erode the patients' trust in the healthcare system, if they believe that they are treated unfairly or that their care is not taken seriously. Healthcare organizations that use biased AI algorithms could also face legal and financial liabilities.

As an example, a care provider may use an AI tool including a trained ML model to predict an effectiveness of a certain treatment for a patient. The ML model may be trained using training data from a training patient population defined by a set of characteristics. For example, the training patient population may comprise primarily white, male patients. The patient may be representative of the training patient population (e.g., a white males), and as a result, the predicted effectiveness of the treatment outputted by the trained ML model may be accurate for the patient. However, the patient may not be representative of the training patient population (e.g., an African-American woman). As a result of not being representative of the training patient population, inferences of the ML model based on patient data may be incorrect, and the predicted effectiveness of the treatment outputted by the trained ML model may not be accurate for the patient. As a result of this algorithmic bias, an inappropriate treatment plan for the patient may be proposed, which may lead to a poorer patient outcome.

In practice, various examples of such algorithmic bias have been discovered. For example, AI models used to predict patient mortality in an intensive care unit (ICU) have been found to be less accurate for women than for men; AI models used to predict the risk of preeclampsia or eclampsia have been found to be less accurate for women of color; AI models used to identify patients at risk of developing sepsis have been found to be biased against African-American patients; AI models used to predict patient risk of hospital readmission have been found to be less accurate for patients with lower socioeconomic status (SES); AI models used to predict patient risk of cardiovascular disease have been found to be less accurate for patients with mobility impairments; as well as others. It should also be appreciated that algorithmic bias may be generally expected of CDPs, given that regulatory approval of CDPs for use is typically based on an evaluation of the CDPs in trials on very limited populations, and a comprehensive assessment of algorithmic bias may not be possible until usage of the CDP on a wider patient population has been established.

Several ways to measure health equity and bias in CDP use have been proposed, such as disparate impact analysis, sensitivity analysis, and monitoring algorithms after they are deployed. Disparate impact analysis involves comparing the outcomes of AI algorithms for different groups of people of different race, gender, socio-economic status, etc. to determine whether there are statistically significant differences. Sensitivity analysis tests the robustness of AI algorithms to changes in the data. For example, an AI algorithm used to predict the risk of readmission to the hospital might be found to be sensitive to changes in the socioeconomic status of a patient.

However, approaches such as these may rely on collecting a large amount of patient outcome data that is linked to specific AI tools or models (e.g., CDPs), which may be cumbersome and time consuming, if not unfeasible. Patient data stored in a hospital database such as an electronic medical record (EMR) system may not include information about tools used by clinicians to make diagnoses or treatment plans that may be associated with patient outcomes. Alternatively, information about the tools or models used by a clinician may be included, but the information may be in a non-standardized format. For example, a CDP may be referenced by name within unstructured text (e.g., a report), where the CDP may not be easily identified as an AI tool. Additionally, a CDP may be referred to differently by different clinicians. For example, a first clinician may reference a CDP by an informal name (e.g., "patient mortality predictor"); a second clinician may refer to the CDP by a company or organization name (e.g., "Siemens mortality predictor"); a third clinician may refer to the CDP by an abbreviation; and so on. Similarly, a first clinician may refer to a patient as Black, a second clinician may refer to the patient as African-American, etc.

As a result, determining whether a CDP might produce different results for different patient populations may entail first retrieving and storing an enormous amount of data from different sources; determining that the data meets diversity thresholds; segregating or classifying the data with respect to various different disparity factors (e.g., race, gender, age, social determinants of health (SDOH), socio-economic status (SES), etc.), and determining a sufficiency of disparity factor data; tracking and aggregating references to the CDP in the data; determining whether treatment of patients was based on an output of the CDP; determining whether a health outcome of the patient was based on the treatment; and encoding and organizing the data (disparity factor classes, outcome classes, etc.) into a standardized format for applying one or more statistical or ML models to evaluate algorithmic bias. Such an endeavor relies on considerable memory and processing resources, which may have to be committed prior to knowing whether sufficient data exists to generate an accurate health equity assessment of the CDP, or when the data will be sufficient.

Moreover, CDPs may be constantly changing. For example, a company that develops an AI model-based predictor of hospital readmission may periodically retrain the AI model, and issue an updated version, or a clinician may add a new rule to a rules-based smart alert CDP. Therefore, a relatively short window of time may be available to perform a reliable health equity assessment of a CDP. For example, a health equity assessment of a CDP using three months of data may be accurate for the next six months, but a health equity assessment of a CDP that takes twelve months may not be accurate by the time it is generated. As a result, using current tools and approaches and currently available data, generating an accurate assessment of a CDP within an appropriate time frame may not be possible.

Thus, a central problem in generating accurate health equity assessments of CDPs used to analyze patient data lies in quickly and efficiently collecting specific data applicable to the assessment process, in a highly standardized format that does not currently exist. This data includes at least disparity factor data (e.g., gender, race, SDOH, SES, etc.) for patients analyzed using the CDP, and simplified/binary data on a performance of the CDP on the patients (e.g., whether an output of the CDP was accurate for the patients or not), that is linked to specific version of a CDP at a specific point in time. By collecting and storing health equity data in the standardized format, rather than sifting through and cross-referencing data already stored in different databases of a healthcare system, the memory and processing resources of healthcare information systems used to analyze the health equity data may be reduced, increasing an efficiency of functioning of the healthcare information systems and increasing an amount of memory and processing resources used for other clinical tasks. Additionally, the memory and processing resources may not be applied until an amount of the health equity data collected is deemed to be sufficient to generate an accurate assessment, reducing a probability of wasted effort. A time spent processing the health equity data may also be reduced, such that suitable assessments may be generated within a short enough window of time to be of use to clinicians, researchers, and product owners, and a development cycle for retraining and/or updating models may be shortened, increasing an effectiveness of the CDPs in practice and promoting increased trust in medical and patient communities.

To address this problem, a confidence and health equity assessment system is provided herein, for CDPs that conform to a common data product definition. In many embodiments, the CDPs may be accessible from a data product marketplace. A user, such as a care provider, may select a CDP available at the data product marketplace via a clinical workflow application or Clinical Information System (CIS). For example, the user may select the CDP from a digital catalog displayed within the clinical workflow application or CIS. The selected CDP may be launched by the user from within the clinical workflow application or CIS. When the CDP is launched, a rating tool micro-application of the confidence and health equity assessment system may be opened within the clinical workflow application and/or the CDP. When the CDP is used for diagnosing or treating a patient, the rating tool micro-application may request clinical feedback from the user regarding the suitability, appropriateness, accuracy, or quality of an output of the CDP via a visual control element displayed on a screen of the user. The clinical feedback may include demographic data with respect to various disparity factors of a patient population analyzed by the CDP. The clinical feedback provided by the user is then aggregated by the confidence and health equity assessment system, which performs a health equity assessment and generates a confidence rating of the CDP, where the confidence rating is multi-dimensional score indicating a degree of confidence that the output of the CDP is not affected by algorithmic bias for various disparity factors. In this way, biases in various CDPs may be identified and rectified, improving the quality and equity of clinical data products and enabling care providers and patients to make more informed decisions. Overall, a greater degree of transparency may be provided with respect to AI model unfairness, which may enable faster adoption of CDPs in standard clinical practice, leading to better patient outcomes.

The confidence and health equity assessment system may rely on various models to generate the health equity assessments. The various models may include AI models, such as a machine learning (ML) or deep learning (DL) models, generative AI or a different kind of model. For example, one or more of the various models may be implemented as a convolutional neural network (CNN) model including a plurality of hidden layers. The models may include classification or prediction models, probabilistic models (e.g., Bayesian models), statistical models, or a different type of model. The various models may also include one or more natural language processing (NLP) models, such as a private implementation of a commercial, open source, or in-house large language model (LLM), such as OpenAI's GPT, which may be trained to provide natural language summaries of outputs of the confidence and health equity assessment system.

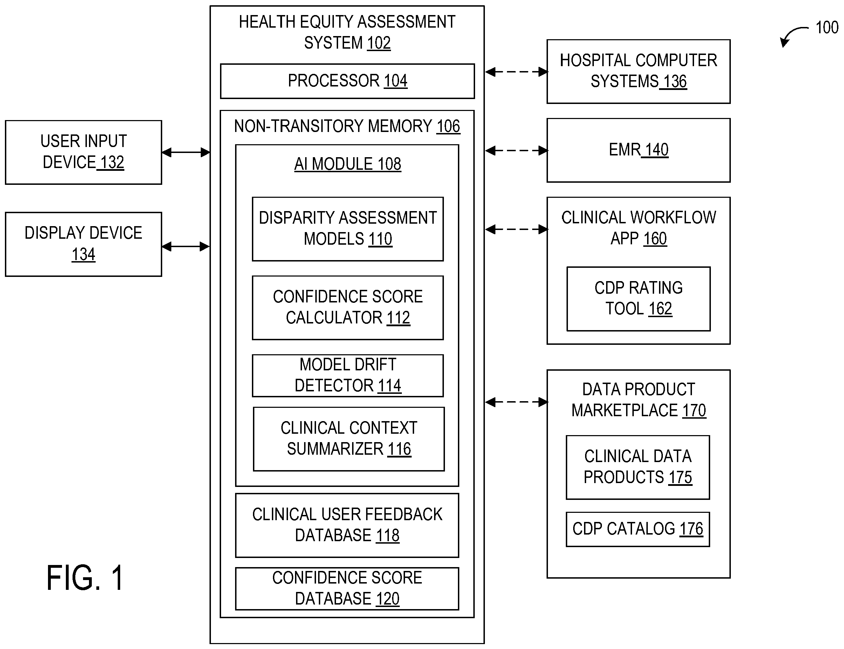

Referring now to the figures, FIG. 1 shows an exemplary health equity assessment system 102, in accordance with an embodiment. As described herein, health equity assessment system 102 may generate an assessment of a confidence of the effectiveness, equity, and sensitivity of results outputted by a CDP used in diagnosing or treating a patient of a healthcare system, with respect to geography, race, age, gender, disease conditions, and/or other factors susceptible to AI algorithm bias.

In some embodiments, at least a portion of health equity assessment system 102 is disposed at a device (e.g., workstation, edge device, server, etc.) communicably coupled to one or more healthcare systems and/or hospital networks and computer systems 136 via wired and/or wireless connections, and can receive or access medical data (including patient data) stored in the one or more healthcare systems and/or hospital computer systems 136. Health equity assessment system 102 may also be operably/communicatively coupled to a user input device 132 and a display device 134. In some examples, user input device 132 may be a shared input device of the one or more healthcare and/or hospital computer systems 136, and display device 134 may be a shared input device of the one or more healthcare and/or hospital computer systems 136.

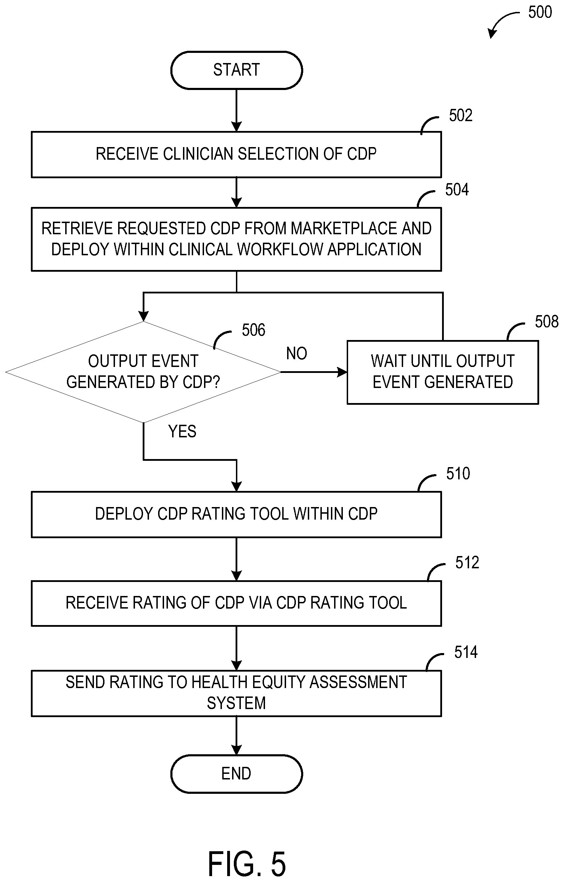

Health equity assessment system 102 may additionally be operably/communicatively coupled to one or more of an electronic medical record (EMR) 140, from which health equity assessment system 102 may retrieve patient records. Health equity assessment system 102 may be operably/communicatively coupled to one or more clinical workflow applications 160 used by a care provider of a healthcare system, for example, when determining diagnostic or treatment options for a patient. In particular, health equity assessment system 102 may receive clinical feedback (e.g., ratings) on an effectiveness of one or more CDPs 175, which may be retrieved from a data product marketplace 170 and launched within the one or more clinical workflow applications 160. The clinical feedback may be communicated to health equity assessment system 102 via a CDP rating tool 162, a micro-application that may be installed in GUI of a clinical workflow application 160 or CDP 175. CDP rating tool 162 is described in greater detail below in reference to FIG. 5.

Data product marketplace 170 may be operably/communicatively coupled to health equity assessment system 102, such that data generated by health equity assessment system 102 may be associated with a CDP 175 of data product marketplace 170. For example, a health equity assessment or rating may be associated with the CDP 175 in a CDP catalog 176 of data product marketplace 170, which may include a list of CDPs 175 that are available for use by the care provider, either as standalone products or within clinical workflow app 160.

Health equity assessment system 102 includes a processor 104 configured to execute machine readable instructions stored in non-transitory memory 106. Processor 104 may be single core or multi-core, and the programs executed thereon may be configured for parallel or distributed processing. In some embodiments, processor 104 may optionally include individual components that are distributed throughout two or more devices, which may be remotely located and/or configured for coordinated processing. In some embodiments, one or more aspects of processor 104 may be virtualized and executed by remotely-accessible networked computing devices configured in a cloud computing configuration.

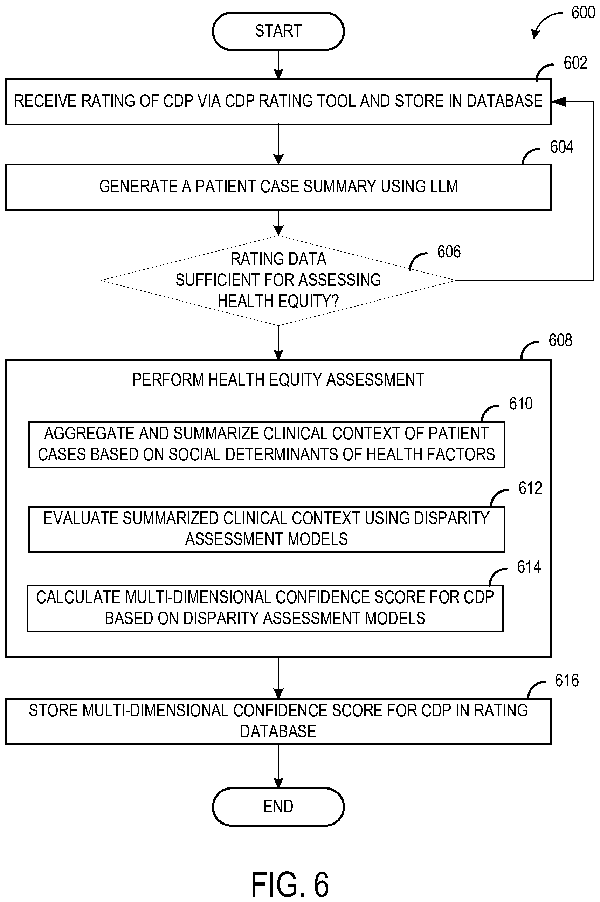

Non-transitory memory 106 may store an AI model module 108, a clinical user feedback database 118, and a confidence score database 120. AI model module 108 may include one or more disparity assessment models 110, a confidence score calculator 112, a model drift detector 114, and a clinical context summarizer 116. AI model module 108 may include various AI models, such as ML and/or DL models. In particular, AI model module may include one or more large language models (LLM) that may be trained to generate a summary of a patient case in natural language, which may be evaluated against the one or more disparity assessment models 110 to determine whether an algorithmic bias exists in a CDP, as described in greater detail below in reference to FIG. 6. In various embodiments, the various AI models and/or disparity assessment models may include neural network models such as convolutional neural networks (CNNs), generative adversarial networks (GAN), transformers and traditional classifier; Bayesian networks, decision trees, and/or other statistical, probabilistic, or hierarchical models. AI model module 108 may include trained and/or untrained neural networks and may further include various data, or metadata pertaining to the one or more AI models stored therein.

The one or more AI models may include confidence score calculator 112, which may calculate a case-specific, multi-dimensional confidence score based on clinical user feedback received from CDP rating tool 162 and stored in clinical user feedback database 118. Confidence scores generated by confidence score calculator 112 for various CDPs may be stored in rating database 118. The confidence scores may be retrieved by CDP catalog 176 and displayed next to listed CDPs 175 of the CDP catalog 176, for example. The confidence scores may be considered by the care provider when selecting a CDP to use with a given patient.

The one or more AI models may include model drift detector 114, which may monitor health equity assessments and confidence ratings of various CDPs, to determine trends in the clinical user feedback. If the clinical user feedback indicates that an overall confidence score of a CDP is decreasing, it may be inferred that an underlying AI model is becoming progressively less accurate due to algorithmic bias (e.g., model drift), whereby a software manufacturer of the CDP may be notified, in one example.

Clinical context summarizer 116 may retrieve social determinants of health disparity factors such as gender, race and ethnicity, socioeconomic status, geographic area, and disability status, among others, from EMR 140 and/or the one or more healthcare and/or hospital computer systems 136, and generate the patient case summary evaluated by the disparity assessment models 110. The patient case summary may be a structured text file, such as a JavaScript Object Notation (JSON) or similar file, which may be formatted for inputting patient data into one or more of the disparity assessment models 110. The patient case summary, or a second patient case summary, may also be structured as a natural language summary, which may be generated by an LLP. The natural language summary may be displayed to clinicians when reviewing CDPs in a CDP catalog, for example.

User input device 132 may comprise one or more of a touchscreen, a keyboard, a mouse, a trackpad, a microphone, a motion sensing camera, or other device configured to enable a user to interact with health equity assessment system 102. In one example, user input device 132 may enable a user to submit a question regarding a patient to health equity assessment system 102 in natural language. For example, the user (e.g., a care provider) may type the question into health equity assessment system 102 using a keyboard, or speak the question into a microphone.

Display device 134 may include one or more display devices utilizing virtually any type of technology. In some embodiments, display device 134 may comprise a computer monitor. Display device 134 may be combined with processor 104, non-transitory memory 106, and/or user input device 132 in a shared enclosure, or may be peripheral display devices and may comprise a monitor, touchscreen, projector, or other display device known in the art, which may enable a user to view responses to queries submitted to health equity assessment system 102, and/or interact with various data stored in non-transitory memory 106.

It should be understood that health equity assessment system 102 shown in FIG. 1 is for illustration, not for limitation. Another appropriate health equity assessment system may include more, fewer, or different components.

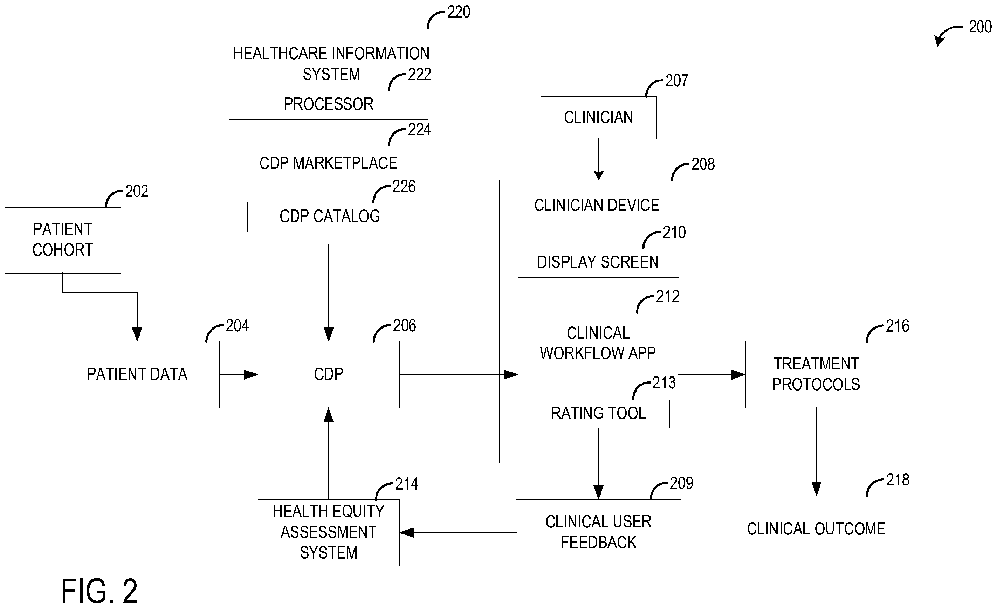

FIG. 2 shows a data flow diagram 200 illustrating how data may be processed by a health equity assessment system 214, which may be a non-limiting example of health equity assessment system 102 of FIG. 1. A clinician 207 (e.g., a care provider of a healthcare system) may wish to analyze patient data from one or more patients of a patient cohort 202 of the healthcare system. Clinician 207 may open a clinical workflow application 212 on a clinician device 208 of clinician 207. For example, the device may be a computer of clinician 207, or a tablet or smart phone of clinician 207, or a different type of computing device. Clinical workflow application 212 may be displayed on a display screen 210 of device 208.

From within clinical workflow application 212, clinician 207 may launch a CDP 206. For example, CDP 206 may be a dashboard that displays patients of patient cohort 202 organized based on patterns in patient data detected by CDP 206, or an AI tool, or a different data analysis product. CDP 206 may be selected by clinician 207 from a CDP marketplace 224, where CDP marketplace 224 may be an online repository of various data analysis products (e.g., CDPs) available for use by clinician 207. CDP marketplace 224 may be hosted within a healthcare information system 220 of the healthcare system, and may include products manufactured by various trusted companies having a business relationship with the healthcare system, for example. In various embodiments, clinician 207 may select CDP 206 from a CDP catalog 226 of the CDP marketplace 224. Healthcare information system 220 includes a processor 222, which may process instructions for managing CDP marketplace 224 and the display of CDPs within CDP catalog 226. In some examples, CDP catalog 226 may be a part of or accessible within clinical workflow app 212.

In various embodiments, clinician 207 may select or define patient cohort 202 using clinical workflow application 212. Clinician 207 may select or define patient data 204 using clinical workflow application 212, or patient data 204 may be selected or defined by CDP 206. CDP 206 may analyze patient data 204, and generate an output that may be displayed on display screen 210. The output may be displayed within a GUI of clinical workflow application 212, or within a GUI of CDP 206, or a different GUI. The output may include predictions, estimations, suggestions, or other results of analytical tools and AI or statistical models applied to patient data 204. From the output, clinician 207 may generate one or more treatment protocols 216 to be applied to patients of patient cohort 202 to achieve a desired clinical outcome 218 for the patients.

To collect data regarding potential algorithmic biases of CDP 206, health equity assessment system 214 may generate and display a rating tool 213 (e.g., CDP rating tool 162 of FIG. 1) on display screen 210. For example, rating tool 213 may be displayed within the GUI of clinical workflow application 212 or the GUI of CDP 206, if applicable. Clinician 207 may use rating tool 213 to indicate a degree of satisfaction of the output of CDP 206 specifically with respect to health equity, e.g., a degree to which whether results produced by CDP 206 are applicable patient cohort 202.

Using rating tool 213, clinical user feedback 209 about the health equity of CDP 206 with respect to patient cohort 202 may be sent to health equity assessment system 214. Clinical user feedback 209 may be stored at health equity assessment system 214 (e.g., in clinical user feedback database 118 of FIG. 1) and processed by health equity assessment system 214 to generate an overall health equity assessment of CDP 206 (e.g., meaning an aggregate health equity assessment over various tasks over time). The health equity assessment may be a multi-dimensional confidence score that CDP 206 does not suffer from algorithmic bias, for example. The overall health equity assessment of CDP 206 may then be stored at health equity assessment system 214 (e.g., in confidence score database 120 of FIG. 1). The overall health equity assessment may be associated with CDP 206 in CDP catalog 226, such that clinicians such as clinician 207 may view the overall health equity assessment of various CDPs listed in CDP catalog 226 prior to selecting an appropriate CDP for a given clinical task.

In some embodiments, CDP catalog 226 may retrieve the overall health equity assessment of various CDPs listed in CDP catalog 226 from health equity assessment system 214 dynamically, in real time, when generating a list of CDPs to display (e.g., in response to search terms used by clinician 207). In other embodiments, the overall health equity assessment of various CDPs listed in CDP catalog 226 may be retrieved and updated periodically from health equity assessment system 214.

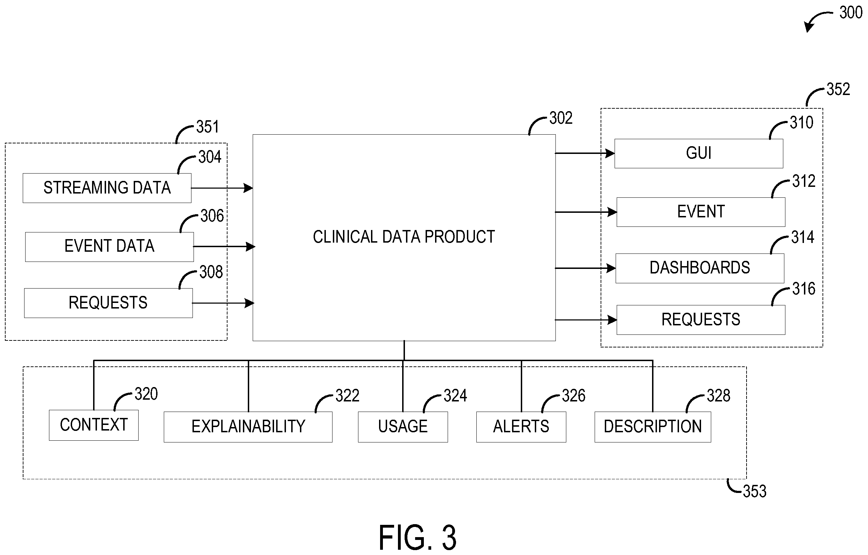

FIG. 3 shows a block schematic diagram 300 illustrating a flow of data through various data ports of a CDP 302 during a use of CDP 302 within the framework of a health equity assessment system, such as health equity assessment system 102 of FIG. 1 and/or health equity assessment system 214 of FIG. 2. CDP 302 may be a non-limiting example of CDP 206. While CDP 302 is described herein as being launched within a clinical workflow application such as clinical workflow application 212, it should be appreciated that in some embodiments, CDP 302 may be used as a standalone product.

For the health equity assessment system to be able to perform a health equity assessment of data outputted by CDP 302, CDP 302 may conform to a common data product definition. The common data product definition may establish a set of input data ports 351 into CDP 302; a set of output data ports 352 from CDP 302; and a set of standardized control data ports 353. In other words, the common data product definition establishes a formal structure that dictates the types of input data that may be received by CDP 302, output data produced by CDP 302 for a clinician, and metadata or contextual data generated from CDP 302 during processing of the input data.

Input data ports 351 may include a streaming data port 304, an event data port 306, and a request port 308. Streaming data port 304 may be used by CDP 302 to receive streaming data, such as, for example, time series data produced by a patient monitor, such as blood pressure, heart rate, ECG, or other vital signs and/or environmental sensor data such as temperature, oxygen, and humidity that may be collected and streamed in real time. CDP 302 may receive event data via event data port 306, where the event data may comprise alerts and alarm data sent by physiological devices and clinical patient data and nursing observations collected over time and stored in a patient health record of an EMR (e.g., EMR 140). Requests transmitted to CDP 302, such as requests from a clinician to perform a specific analysis or generate a specific report or result, may be received via requests port 308.

Output data ports 352 may include a GUI port 310, an output event port 312, a dashboard port 314, and an output request port 316. Result data generated by CDP 302 based on input data received at input data ports 351 may outputted via GUI port 310 to be displayed in a GUI, such as a GUI of the CDP 302 or a GUI of the clinical workflow application. Similarly, result data generated by CDP 302 based on input data received at input data ports 351 may outputted via dashboards port 314 to be displayed in a dashboard, such as a dashboard of the clinical workflow application. For example, the dashboard data may be organized in a spatial layout in columns and/or rows, and may include control elements for performing actions or accessing additional data (e.g., drilling down). Event data generated by CDP 302 may be outputted via output event port 312. The event data may comprise data that may be inputted into a different software component or tool for further analysis, for example.

In some cases, CDP 302 may generate requests for data from other sources, such as the EMR or other clinical databases accessible on hospital networks (e.g., hospital computer systems 136) or within hospital information systems (e.g., healthcare information system 220). For example, a first algorithm of CDP 302 may perform a first analysis of patient data to generate a first result. Based on the first result, a request may be generated for additional information for processing by a second algorithm of CDP 302, which may be outputted via output requests port 316. The requested data may be retrieved and received by CDP 302 via input event port 306, and processed by the second algorithm. A result of the second algorithm may be outputted to a GUI via GUI port 310, or to a dashboard via dashboards port 314.

Standardized control data ports 353 may include a context port 320, an explainability port 322, a usage port 324, an alerts port 326, and a description port 328. Context port 320 may include patient/clinical context data passed via the input data to the CDP (e.g. patient identifiers such as medical record number, SSN, encounter date, location, department, etc.), which may enable an unambiguous look up of additional patient demographics and SDOH information from an EMR.

Data for healthcare AI models that passes through explainability port 322 may take various forms depending upon a specific model type and desired level of interpretability. For example, feature attribution data such as saliency maps, LIME (Local Interpretable Model Agnostic Explanations), SHAP (Shapley Additive exPlanations), or other feature attribution data may be included. The data may be generated during the AI model inferencing.

Data that passes through usage port 324 may provide information about a clinical workflow application where an AI model was used. This data may be static data that is embedded within CDP 302 when CDP 302 is deployed within a clinical workflow.

The alerts port 326 allows contextual information to be collected about clinical alerts, alarms, notifications, etc. that might have been triggered by an AI model. The contextual information may be generated during the AI model inferencing.

The description port 328 may allow for a human readable description (e.g., static text) of a purpose of CDP 302 to be collected, including one or more embedded AI models of CDP 302.

It should be appreciated that the examples described above are for illustrative purposes, and in other embodiments, a greater or lesser number of ports may be defined for CDP 302, or different types of ports, without departing from the scope of this disclosure. For example, in some embodiments, standardized control data ports 353 may include an AI model output performance metrics port, a data drift distribution (e.g., input features) port to enable an AI model drift assessment as described in greater detail below, and/or other types of ports.

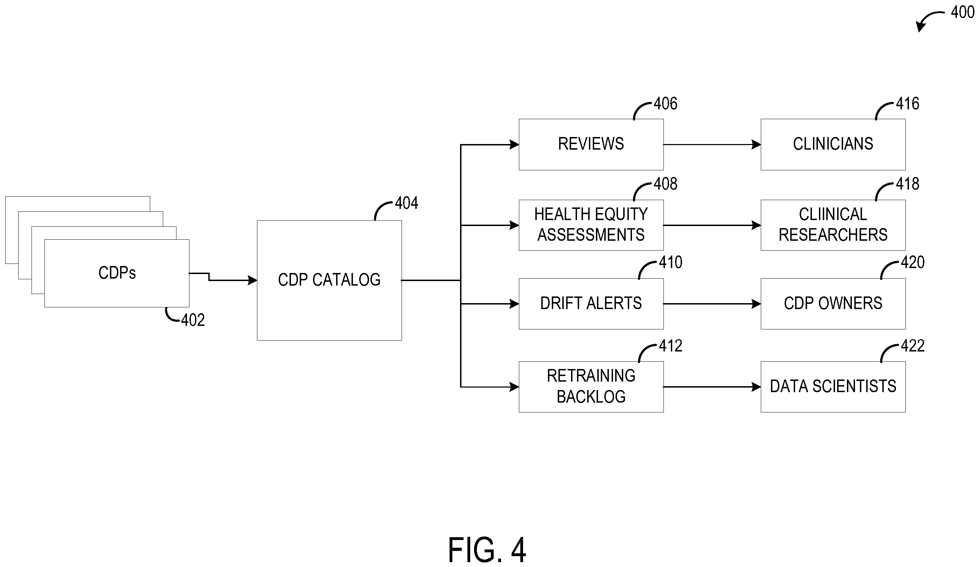

FIG. 4 shows an exemplary data product marketplace 400, which may be the same as or similar to the data product marketplace 170 of FIG. 1. Data product marketplace 400 includes a plurality of CDPs 402 (e.g., CDPs 206), which may be selected by a clinician for analyzing patient data. In various embodiments, the CDPs 402 may be listed in a CDP catalog 404 (e.g., CDP catalog 226), where clinicians 416 may view or search for CDPs that may be applied to patient data for a desired clinical task. In addition to the clinicians 416, users of CDP catalog 404 and the CDPs 402 included therein may include clinical researchers 418, CDP owners 420, and data scientists 422, among others.

As described above, CDP catalog 404 may be created and hosted by a hospital information system such as healthcare information system 220. CDP catalog 404 may be displayed within a GUI of the hospital information system, or CDP catalog 404 and may be retrieved from the hospital information system and displayed within a clinical workflow application (e.g., clinical workflow application 212) used by the clinicians 416. In other embodiments, CDP catalog 404 may be generated by clinical workflow application. In still other embodiments, CDP catalog 404 may be created by the health equity assessment system, and displayed on a display device (e.g., display device 134) of the health equity assessment system, or retrieved from the health equity assessment system and displayed in the GUI of the clinical workflow application.

For each CDP 402 listed in CDP catalog 404, data about the CDP may be displayed or made accessible to (e.g., linked) a user of CDP catalog 404. A first portion of the data may be displayed in a top-level view, meaning that some data of each CDP 402 may be displayed next to the CDP 402 on the list, to aid a user in selecting a suitable CDP 402 for a desired task. The first portion of the data may include, for example, an intended use of the CDP 402, a result or benefit that may be expected from analyzing the patient data using the CDP 402, an intended patient population of the CDP 402, etc. A second portion of the data may be displayed when a CDP 402 is selected from the list. The second portion of the data may include, for example, one or more reviews 406 of the CDP 402; a health equity assessment 408 of the CDP 402; a drift alert 410 of the CDP 402; and/or a retraining backlog 412 of the CDP 402.

Different elements of the second portion of CDP data may be relevant to the different types of users of the CDPs 402. As a first example use case, a clinician 416 may scan through the list of available CDPs 402 in CDP catalog 404, using CDP data of the first portion to identify candidate CDPs for a desired clinical analysis. The clinician 416 may select a CDP 402 in the CDP listing of CDP catalog 404, which may display a page or view with additional information of the second portion. The additional information may include a review of the CDP 402. The clinician 416 may read the review to determine a potential effectiveness of the CDP 402 for the desired clinical analysis. The clinician 416 may also view a health equity assessment of the CDP 402, to assess a possibility that the CDP 402 may have been created or trained based on data from a training patient population that may not match a patient cohort (e.g., patient cohort 202) of the desired clinical analysis. If the health equity assessment leads the clinician 416 to believe that the training patient population may not match the patient cohort, the clinician 416 may select a different CDP 402 for the desired clinical analysis.

In some embodiments, the health equity assessment may comprise a multi-dimensional rating or score for the CDP 402, where each dimension of the multi-dimensional rating or score may correspond to a disparity factor. For example, the multi-dimensional rating may include a first rating for gender disparity; a second rating for racial disparity; a third rating for disparities related to disability; a fourth rating for SDOH; a fifth rating for language barriers; and so on. In this way, an algorithmic bias of CDP 402 may be assessed and indicated separately for each disparity factor.

In some embodiments, the health equity assessment may be stored in CDP catalog 404, or stored in CDP 402. In other embodiments, the clinician 416 may select a health equity assessment control element in CDP catalog 404 or in the page or view with additional information, and the health equity assessment may be retrieved from a database (e.g., confidence score database 120) of the health equity assessment system and displayed in the page or view with additional information. For example, the health equity assessment may be retrieved in real time.

As a second example use case, a clinical researcher 418 may be performing research on the health equity of CDPs available on CDP marketplace 400. The clinical researcher 418 may review various health equity assessments 408 of various CDPs 402, and compare the various health equity assessments 408, integrate the various health equity assessments 408 into other research, and/or view other data associated with the various CDPs 402 to confirm or further clarify the health equity assessments 408.

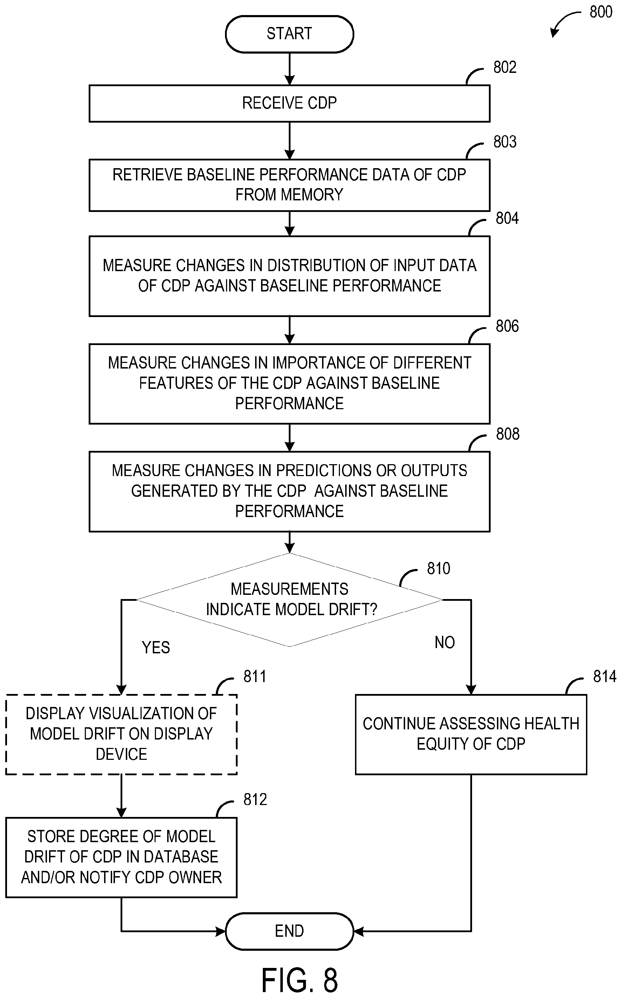

As a third example use case, a CDP owner 420 may review a CDP 402 owned by CDP owner 420 to determine whether a drift alert 410 may be associated with the CDP 402. The drift alert 410 may be generated by a model drift detector (e.g., model drift detector 114 of FIG. 1) of the health equity assessment system. In some embodiments, the model drift detector may monitor drift in CDP models, and automatically generate the drift alert 410 in response to detecting a decrease in accuracy, performance, or effectiveness of the CDP 402 over time, as the CDP 402 is applied to diverse patient populations. In response to a drift alert, the CDP owner 420 may decide to remove the CDP 402 from CDP catalog 404, and/or notify one or more data scientists 422 responsible for the CDP 402 to perform a retraining of one or more models of the CDP 402. The generation of drift alerts is described in greater detail below in reference to FIG. 8.

Additionally, CDP owner 420 may review ratings of CDP products and decide how and where to package the CDP products to various digital applications or solutions with respect to use case applicability in light of potential biases discovered. For example, a CDP may be recommended for a first patient population for which algorithmic bias was not found, and the CDP may not be recommended for a second patient population for which algorithmic bias was detected. For example, the CDP may be recommended for patients within an age range, or not recommended for patients taking certain medications, etc.

As a fourth example use case, a data scientist 422 may consult a retraining backlog 412 of a CDP 402. In various embodiments, retraining backlog 412 may be a collection of clinical user feedback stored in reference to CDP 402. For example, in some embodiments, when providing a rating of a CDP 402, a clinician may also enter in written feedback that may be stored at the health equity assessment system. Written feedback from various clinicians may accumulate over time into the retraining backlog 412. The data scientist 422 may use the retraining backlog 412 of accumulated feedback to inform further training of the CDP 402. For example, the accumulated feedback may indicate that an AI model of the CDP 402 performs poorly on patients of a certain demographic group, and the data scientist 422 may retrain the AI model on a training dataset compiled from patients of the demographic group to increase an overall accuracy of the AI model and/or reduce an algorithmic bias of the AI model.

Referring now to FIG. 5, a method 500 is shown for capturing clinical feedback with respect to a CDP, using a CDP rating tool micro-application (e.g., CDP rating tool 162 of FIG. 1) that may be installed within a clinical workflow application, such as clinical workflow application 212 of FIG. 2. Method 500 may be performed by a processor of a computing device of a care provider on which the clinical workflow application runs. Portions of method 500 may be performed based on instructions stored in the CDP rating tool micro-application and executed by the processor. The clinical feedback may subsequently be processed by a health equity assessment system such as health equity assessment system 102 of FIG. 1 and/or health equity assessment system 214 of FIG. 2, to assess whether algorithmic bias may be affecting an output of the CDP. The processing of the clinical feedback by the health equity assessment system is described below in reference to FIG. 6.

Method 500 begins at 502, where method 500 includes receiving a clinician selection of a CDP. The clinician may select a CDP from a catalog of data products, as described above, based on a clinical task desired to be performed with respect to a patient of the clinician.

At 504, method 500 includes retrieving the selected CDP from a repository of CDPs linked to from the CDP catalog, and deploying the selected CDP within the clinical workflow application. When the selected CDP is deployed, the CDP may receive input data including data of the patient at one or more input ports of the CDP (e.g., input ports 351), as specified by the clinician via a GUI of the CDP or clinical workflow application. The CDP may perform a predetermined analysis of the input data, and output a result via one or more output ports of the CDP (e.g., output ports 352).

At 506, method 500 includes determining whether an output event is generated by the CDP. The output event may be generated when a result of the analysis performed by the CDP on the patient data is available. The result may include an output of an AI model, such as an ML model, or a statistical model, or the result of a rules-based system such as a decision tree. For example, the result may be an alert, or a possible diagnosis, or a suggestion for a proposed treatment, or a different type of result.

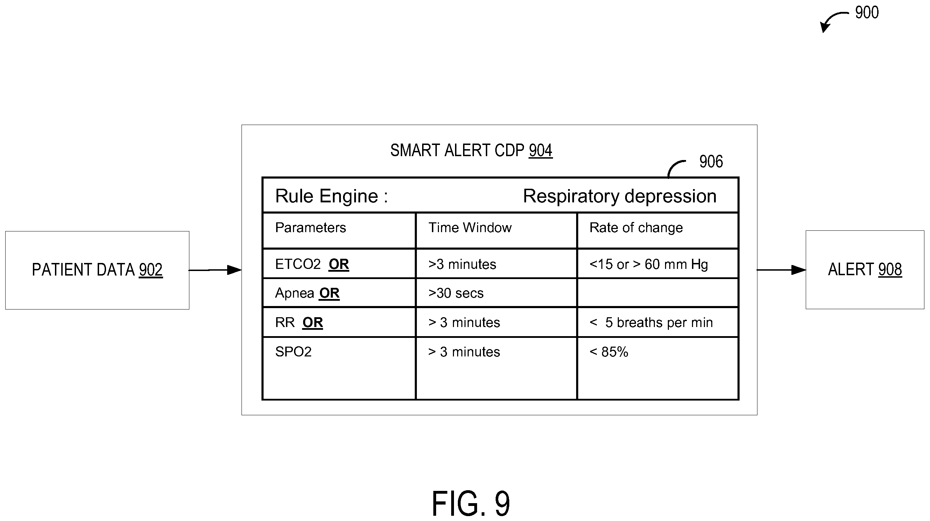

Referring briefly to FIG. 9, a CDP event generation diagram 900 shows an exemplary CDP 904 that may be deployed within the clinical workflow application as described in method 500. Exemplary CDP 904 is a smart alert data product that may generate an alert 908 in response to one or more conditions being met by a set of patient data 902 of a patient. In the depicted embodiment, CDP 904 is a smart alert related to respiratory depression of the patient, where alert 908 may be generated in response to a set of conditions established within a rule engine 906 of CDP 904. Alert 908 may be displayed within a GUI of the clinical workflow application, or transmitted to a care provider that set up the smart alert in a different manner.

CDP 904 may be created by the care provider, where the set of conditions established in rule engine 906 may be established by the care provider. Alternatively, CDP 904 may be predefined with the set of conditions and selected via a CDP catalog, as described in reference to method 500.

In the depicted embodiment, the set of conditions include various parameters, and corresponding target values or thresholds. For example, the set of conditions of rule engine 906 may establish that if an end-tidal CO₂ of the patient measured over at least three minutes is below 15 mm Hg or above 60 mm Hg, or an apnea of the patient lasts longer than 30 seconds, or a respiratory rate of the patient measured over at least three minutes is below 5 breaths per minute, or an oximeter reading of the patient over at least three minutes is below 85%, then alert 908 may be generated. It should be appreciated that CDP 904 is one example of a CDP, and other examples may include a fewer or greater number of conditions, and/or conditions of different types, without departing from the scope of this disclosure. Furthermore, in other embodiments, CDP 904 may be a different type of model that generates a different type of output.

Returning to method 500, if at 506 it is determined that an output event has not been generated by the CDP, method 500 proceeds to 508. At 508, method 500 includes waiting until an output event is generated, and method 500 proceeds back to 506. Some time may pass before the output event is generated, or in the case of alerts, the output event may not be generated.

Alternatively, if at 506 it is determined that an output event has been generated by the CDP, method 500 proceeds to 510. At 510, method 500 includes deploying the CDP rating tool within the GUI of the CDP or GUI of the clinical workflow application. In other words, the CDP rating tool may be automatically deployed in response to the event being generated by the CDP. In one embodiment, when the CDP rating tool is deployed, a set of control elements (e.g., selectable icons) may be displayed that allows the clinician to provide clinical feedback to the health equity assessment system. The clinical feedback may be provided with a single selection action, such as a click of a mouse or similar input device, after the clinician has reviewed the output event.

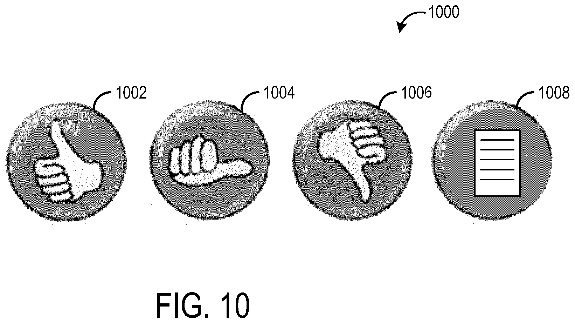

Referring briefly to FIG. 10, an exemplary set of control elements 1000 is shown, which may be displayed in a GUI of a CDP or clinical workflow application in which the CDP is used. For example, the set of control elements 1000 may be superimposed on a portion of the GUI, such as next to an indication of a result outputted by the CDP. The set of control elements 1000 includes a first control element 1002, a second control element 1004, a third control element 1006, and a fourth control element 1008. First control element 1002 includes a "thumbs up" icon, where selecting first control element 1002 may indicate that the output of the CDP was correct, or accurate, or that the clinician finds no evidence that the output of the CDP may be biased towards a patient population different from the patient or cohort of patients (e.g., patient cohort 202) being rated by the clinician. Third control element 1006 includes a "thumbs down" icon, where selecting third control element 1006 may indicate that the output of the CDP was not correct, or inaccurate, or that the clinician believes that the output of the CDP may be biased towards a patient population different from the patient or cohort of patients. Second control element 1004 includes a "thumbs sideways" icon, where selecting second control element 1004 may indicate that the clinician does not have sufficient information to assess a potential bias of the output of the CDP. Fourth control element includes a "note" icon, where selecting fourth control element 1008 may open an editor pane that may allow the clinician to enter in comments or written feedback to be collected by the health equity assessment system. For example, the written feedback may be used to generate the retraining backlog 412 described above in reference to FIG. 4. In this way, the clinician may quickly and efficiently rate the output of the CDP within a normal workflow of the clinician and without being distracted or burdened by opening and closing additional screens, entering in information, or navigating various control options.

It should be noted that in other embodiments, the set of control elements 1000 of the CDP rating tool may appear differently, and may include different icons, images, or symbols, and may include a greater or fewer number of options.

Returning to method 500, at 512, method 500 includes receiving a health equity rating of the CDP via the CDP rating tool, for example, in the manner described above. When the clinician selects one of the control elements 1002, 1004, or 1006, all of the control elements 1002, 1004, or 1006 may disappear from the display. In some examples, the CDP rating tool may additionally be displayed via a menu of the clinical workflow application or CDP GUI, such that the clinician may change their feedback in the event of a mistake. The rating of the CDP may be accompanied by written feedback entered in via fourth control element 1008, or no written feedback may be provided.

At 514, method 500 includes sending the clinical feedback (e.g., the rating generated by the CDP rating tool) to the health equity assessment system, and method 500 ends. In various embodiments, the clinical feedback may be sent to the health equity assessment system via a hospital network (e.g., hospital computer systems 136), or via the Internet, or via a different private or public network.

Referring now to FIG. 6, a method 600 is shown for generating a health equity assessment of a CDP, using a health equity assessment system such as health equity assessment system 102 of FIG. 1. Method 600 may be a machine-implemented method performed by a processor of the health equity assessment system (e.g., processor 104), based on instructions stored in a non-transitory memory of the health equity assessment system (e.g., non-transitory memory 106). The health equity assessment may be generated based on ratings provided by clinicians, as described above in reference to method 500 of FIG. 5.

Method 600 begins at 602, where method 600 includes receiving a rating of a CDP being used by a clinician to analyze data of a patient, where the rating may be sent via a CDP rating tool as described above. The rating may include an indication of a degree of satisfaction of the clinician with respect to an output of the CDP. Specifically, the rating may indicate whether the clinician believes the output to be accurate for the patient; whether the clinician believes the output not to be accurate for the patient, potentially as a result of algorithmic bias of a model of the CDP; or whether the clinician lacks sufficient information to form a judgment regarding potential algorithmic bias of model of the CDP. The rating may also include written feedback provided by the clinician. The rating (and written feedback, if included) may be stored in a database of the health equity assessment system (e.g., clinical user feedback database 118 of FIG. 1). The rating and written feedback may be aggregated with other ratings and written feedback provided by other clinicians.

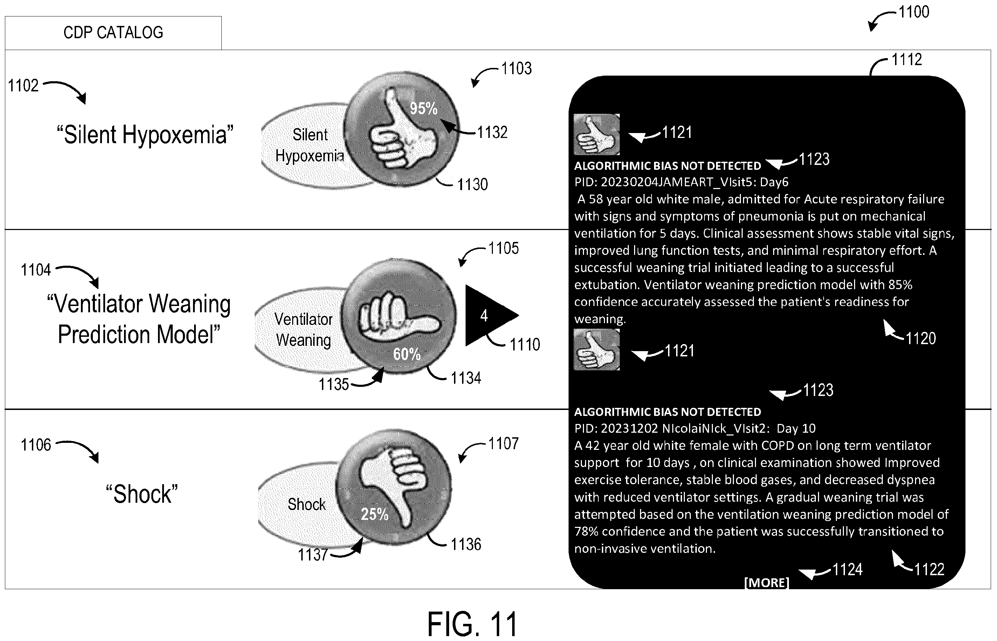

At 604, method 600 includes generating a patient case summary of the usage of the CDP being rated with respect to a patient or cohort of patients. The patient case summary may rely on a clinical context summarizer model or module of the health equity assessment system (e.g., clinical context summarizer 116), which may retrieve social determinants of health factors (e.g., health disparity factors) of the one or more patients, such as, for example, a racial or ethnic background of the patient or cohort of patients; a gender of the patient or cohort of patients; an age of the patient or cohort of patients; and/or other factors that may affect an accuracy of an output of the CDP. In various embodiments, the patient case summary may be generated as a natural language (e.g., unstructured text) summary using an LLM. For example, the LLM may be prompted to search patient records of an EMR (e.g., EMR 140) and retrieve clinical context data including a patient condition, diagnoses, treatments, and the health disparity factors, and to generate a summary report for future review by clinicians. The natural language summary may be stored in a database of the health equity assessment system, from which it may be retrieved and included in health equity assessment information provided about the CDP, for example, in a CDP catalog (e.g., CDP catalog 226). An example of the natural language summary is described below in reference to FIG. 11.

At 606, method 600 includes determining whether a sufficient amount of rating data has been collected to perform a health equity assessment of the CDP. In some examples, a health equity assessment may be performed each time a rating is received, and a result of the health equity assessment may be associated with the CDP based on one rating or a small amount of rating data. In other examples, the health equity assessment may not be performed each time a rating is received, and the health equity assessment may be performed after a predetermined threshold amount of rating data has been received from the clinician or from a plurality of clinicians. The predetermined threshold amount of rating data may be established in various ways and in accordance with various criteria, and may vary depending on a type of CDP, or a risk associated with acting on an output of the CDP, or other factors, for example. In some cases, the health equity assessment may be performed when the collected rating data includes a threshold number of negative ratings indicating a possible algorithmic bias of the CDP.

If at 606 it is determined that the rating data is not sufficient to perform the health equity assessment of the CDP, method 600 proceeds back to 602, where method 600 waits to receive additional ratings. Alternatively, if at 606 it is determined that the rating data is sufficient to perform the health equity assessment of the CDP, method 600 proceeds to 608.

At 608, method 600 includes performing a health equity assessment of the CDP, based on the aggregate rating data collected from one or more clinicians and/or users of the rating tool. Performing the health equity assessment of the CDP includes, at 610, aggregating and summarizing the clinical contexts of each use of the CDP on patients for whom rating data has been collected. The clinical context may include the social determinants of health factors (e.g., health disparity factors) of the one or more patients described above. The clinical context may be summarized by the clinical context summarizer of the health equity assessment system. In one embodiment, the clinical context summarizer may include an AI model, such as a rules-based system or a ML model, which may summarize the clinical context into a structured text document or file such as a JSON file. The structured text document may include labeled data in a standardized format, which may be used as input data for a disparity assessment model. In some examples, the clinical context summarizer may rely on an LLM to generate the structured text document, and/or may rely on patient case summaries generated by an LLM as described above to generate the structured text document.

At 612, performing the health equity assessment includes evaluating the summarized clinical context data using one or more disparity assessment models. The disparity assessment models may take data from the structured text document, and analyze or process the summarized clinical context data to determine a degree to which an output of the CDP, as rated by clinicians, may be affected by algorithmic bias with respect to one or more of the disparity factors. The disparity assessment models may assess the output of the CDP in accordance with various metrics and/or techniques. For example, the disparity assessment models may assess an accuracy, precision, or sensitivity of the CDP. The disparity assessment models may use metrics such as calculating an F 1 score; calculating a mean squared error (MAE) or root mean squared error (RMSE); calculating an R-squared value; calculating an area under a receiver operating characteristic (ROC) curve; or a different metric.

For example, a first disparity model may comprise a statistical model that analyzes correlations between ratings and different sub-groups of a patient population analyzed using the CDP, using various statistical techniques known in the art. A second disparity model may comprise a convolutional neural network (CNN) that is trained to predict a degree of algorithmic bias in the summarized clinical context data. In general, there may be various types of health disparity models, each focusing on different aspects of the complex issue of unequal health outcomes across populations. Examples may include conceptual models, statistical models, and/or other types of models.

The conceptual models may include, for example:
- Social Determinants of Health (SDOH) Model: This model views social and economic factors like income, education, and housing as fundamental determinants of health, creating inequities in access to healthcare, quality of life, and ultimately, health outcomes.
- Intersectional Model: This model highlights the complex interplay of multiple identities and social positions (e.g., race, gender, socioeconomic status) in shaping health experiences and disparities.
- Equity-Targeted Intervention Model: These interventions are specifically designed to address the needs and challenges of populations experiencing health disparities, promoting targeted resource allocation and culturally appropriate strategies.

Examples of the statistical models may include, for example:
- Causal Inference Models: These models attempt to establish causal relationships between specific interventions or policies and changes in health disparities, guiding effective interventions.
- Machine Learning Models: These models can be used to identify patterns and associations in complex health data, potentially revealing previously unknown factors contributing to disparities.
It should be appreciated that the examples described herein are for illustrative purposes, and various different types of disparity assessment models known in the art may be used to assess the health equity of the CDP without departing from the scope of this disclosure.

In some embodiments, the disparity assessment models may include or be used in conjunction with a model drift detector (e.g., model drift detector 114), to detect changes in the performance of a CDP over time. Various metrics may be used to measure model drift.

Referring briefly to FIG. 8, a method 800 is shown for assessing model drift in a CDP. Before a CDP is deployed and made available in the marketplace, a baseline performance of the CDP may be assessed against an initial training dataset, such that future performance of the CDP may be measured against the baseline performance. The baseline performance data may include, for example, key metrics such as model accuracy, precision, recall, F1-score, area under a receiver operating characteristic curve (AUC-ROC), and/or other metrics of the metrics and methods described below. The key metrics used in the baseline performance and a statistical distribution of model input features may be collected and stored in a memory of the health equity assessment system, such as non-transitory memory 106 of FIG. 1.

After the CDP is deployed to clinical environments for use, the same key metrics as used in the baseline performance assessment may be tracked periodically or continuously automatically by a model drift detector (e.g., model drift detector 114) on new unseen data. Similarly, the statistical distribution of model input features in real world data may be continuously tracked. Drift in the data may be periodically assessed using one or more steps of method 800. It should be appreciated that in different embodiments, one or more steps of method 800 may be performed in a different order than described below, and/or additional steps may be added. Method 800 may be performed by a processor of the health equity assessment system, such as processor 104 of FIG. 1.

Method 800 begins at 802, where method 800 includes receiving a CDP to be assessed with respect to model drift. The CDP may be a CDP that is rated by a clinician, as described above in relation to methods 500 and 600.

At 803, method 800 includes retrieving the baseline performance data of the CDP from a memory of the health equity assessment system.

At 804, method 800 includes measuring changes in a distribution of input data used by the CDP over time, using various methods. For example, the methods may include one or more distance metrics, such as the following:
- Kolmogorov-Smirnov (KS) Test: Compares the cumulative distribution functions of two datasets, where a larger KS statistic indicates greater drift.
- Earth Mover's Distance (EMD): Measures the minimum cost of transforming one distribution into another, where a higher EMD suggests more significant drift.
- Jensen-Shannon Divergence (JSD): Measures the information divergence between two probability distributions, where a higher JSD signifies greater difference.

The various methods may include one or more statistical tests, such as the following:
- Chi-squared Test: Compares the observed and expected frequencies of categorical variables in two datasets, where a significant p-value indicates drift.
- Wilcoxon signed-rank test: Compares paired samples from two populations to see if their medians differ, where a large statistic suggests drift.
- Population Stability Index (PSI): Measures the change in distribution of individual features between two datasets, where a higher PSI values indicate larger drift.

The various methods may include one or more model-based approaches, such as the following:
- Residual analysis: Analyzes the difference between the model's predictions and actual outcomes. Increasing residuals over time may signal drift.
- Change point detection algorithms: Identifies statistically significant shifts in model performance over time.
- Drift detectors based on anomaly detection: A separate model may be trained to identify anomalous data points that might indicate drift.

At 806, method 800 includes measuring changes in a relative importance of different features of the CDP over time. Data drift occurs when a first distribution of input data to an AI model in production significantly differs from a second distribution of data used for training. This can lead to inaccurate predictions and model degradation over time. Methods for measuring input feature changes include:
- Statistical Tests by comparing the distributions of input features between training and production data using for test such as Kolmogorov-Smirnov test (KS test), Wasserstein distance and Jensen-Shannon divergence (JSD).
- Feature Importance Analysis - the features most influential on model predictions are established during training to be monitored closely for drift during production.
- Monitoring Data Ranges and Distributions tracking statistical measures (mean, standard deviation, quantiles) over time and visualize distributions for comparison.
- Concept Drift Detection Algorithms - machine learning algorithms and toolboxes specifically designed to detect changes in data distributions such as Adaptive Windowing (ADWIN), Drift Diffusion Model (DDM), Hierarchical Bayesian estimation of the Drift Diffusion Model (HDDM), etc.

At 808, method 800 includes measuring changes in predictions or outputs of the CDP over time. Various methods may be used to measure changes in AI model outputs, predictions, and performance over time on production data, such as:
- Tracking Performance Metrics - Mean squared error (MSE), mean absolute error (MAE), R-squared for regression tasks and AUC-ROC for binary classification.
- Monitoring Prediction Distributions - track the distribution of model predictions over time to detect shifts in prediction patterns that might indicate drift. Common metrics include Population Stability Index (PSI), Kolmogorov-Smirnov (KS) test, and Histogram comparisons.
- Analyzing Error Distributions - the distribution of prediction errors may be analyzed to identify systematic biases or changes in error patterns.
- Using Concept Drift Detection Techniques and Tracking of Input Feature Changes: algorithms specifically designed to detect changes in data distributions or relationships between features and target variables. Examples: ADWIN, DDM, HDDM.
- Monitoring Model Confidence based on the User Feedback - tracks model confidence scores to detect changes in uncertainty, where a decreasing confidence score may indicate drift of model uncertainty.

At 810, method 800 includes determining whether the measurements made at steps 804-808 indicate that an underlying model of the CDP has drifted more than a threshold permissible amount of drift. In various embodiments, drift can be expressed as a numeric score and thresholds can be set accordingly. The drift score may serve as a quantitative indicator of change in a model's underlying data or behavior. For example, the drift score may be distribution-based, or performance-based.

The distribution-based scores measure the difference between the distributions of input features or model outputs in training and production data, and may include the following:
- Kolmogorov-Smirnov (KS) Statistic: This quantifies the maximum difference between the cumulative distribution functions of two datasets. Values range from 0 to 1, with higher values indicating greater discrepancies. KS Scores exceeding 0.3 generally indicate a significant difference between the distributions pointing to a potential impactful drift.
- Population Stability Index (PSI): This calculates the expected relative frequency of categories or values in one distribution compared to another. PSI < 0.1: no significant drift, PSI < 0.2: moderate drift, PSI > 0.2 for significant drift.
- Earth Mover's Distance (EMD): This measures the minimum cost of transforming one distribution into another, offering a more nuanced comparison than KS. The EMD scores might be monitored over time and compared against the baseline score. Relative EMD values close to zero indicate highly similar distributions and EMD values exceeding 1 potentially indicating impactful drift.

The performance-based scores track changes in the model's performance metrics based on new data encountered in production, and may include the following:
- Change in Accuracy, Precision, Recall, F1-score, AUC-ROC: Monitoring absolute or relative changes in these metrics over time can signal potential drift.
- Mean Squared Error (MSE), Root Mean Squared Error (RMSE): These error metrics can reveal performance deviations for regression models.
- Quantile Loss: Evaluating changes in quantiles of the predicted error distribution helps identify shifts in prediction behavior.

If at 810 it is determined that the underlying model of the CDP does not show signs of drift, method 800 proceeds to 814. At 814, method 800 includes continuing to assess the health equity of the CDP, and method 800 ends. Alternatively, if at 810 it is determined that the underlying model of the CDP has drifted more than the threshold permissible amount of drift, method 800 proceeds to 811.

At 811, method 800 optionally includes displaying the model drift on a display device. For example, the drift score, a textual description of the drift, and/or one or more visualizations of the drift may be displayed. The display device may be a display device of the health equity assessment system (e.g., display device 134), or the model drift may be displayed within a listing of the CDP in a CDP catalog (e.g., CDP catalog 404) on a display device of a personal computing device of an owner of the CDP, a clinician, a researcher, a data scientist, or a different individual. The visualizations may be generated using one or more visualization techniques, such as the following, as a non-limiting list:
- Histograms: Compares the shapes and spreads of feature distributions visually. Overlapping or shifted histograms suggest drift.
- Boxplots: Compares the medians, quartiles, and outliers of feature distributions in two datasets. Boxplots with significant differences indicate drift.
- Time series plots: Plot key metrics like accuracy or error over time to visually identify trends and sudden changes that might suggest drift.

At 812, method 800 includes storing the assessed degree of model drift in a database of the health equity assessment system, and/or notifying an owner of the CDP and/or other stakeholders in the health equity assessment system, and method 800 ends.

Returning to method 600, at 614, performing the health equity assessment includes calculating a multi-dimensional confidence score for the CDP, based on outputs of the one or more disparity assessment models. Each dimension of the multi-dimensional confidence score may correspond to a health disparity factor.

As an example, in one embodiment, input data may be extracted from the structured text document and inputted into an input layer of a CNN, and the CNN may output confidence scores for one or more disparity factors, where each confidence score indicates a degree of confidence (for example, on a scale of one to 10) that the output of the CDP does not suffer from algorithmic bias with respect to a corresponding disparity factor. For example, the CNN may output a first confidence score of nine for the disparity factor "gender", indicating a high degree of confidence that outputs of the CDP are not biased towards a given gender. The CNN may output a second confidence score of six for the disparity factor "race", indicating a lower degree of confidence that the outputs of the CDP are not biased towards patients of a given race (e.g., that algorithmic bias exists). The CNN may output a third confidence score of 10 for the disparity factor "age", indicating a high degree of confidence that outputs of the CDP are not biased towards patients of a certain age range; and so on. In this way, the multi-dimensional confidence score may comprise the various confidence scores outputted by the CNN.

In other embodiments, different dimensions (e.g., confidence scores) of the multi-dimensional confidence score may be generated by different disparity assessment models. That is, a first model may be used to generate a confidence score regarding algorithmic bias with respect to race; a second model may be used to generate a confidence score regarding algorithmic bias with respect to gender; a third model may be used to generate a confidence score regarding algorithmic bias with respect to SES; and so on. The first, second, and third models may be different models or types of models, or may include the same models or types of models. For example, the first model may be a first statistical model; the second model may be a CNN; the third model may be a second statistical model; etc.

Further, in some embodiments, various models may be applied to assess algorithmic bias with respect to a single disparity factor, and an output (e.g., confidence score) of a best-performing model may be selected. Alternatively, a confidence score for the disparity factor may be generated based on a consensus between the outputs of the various models.

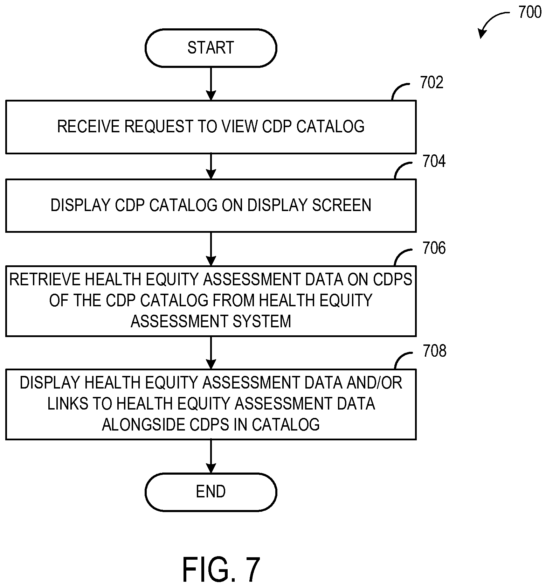

At 616, method 600 includes storing the multi-dimensional confidence score for the CDP in a database of the health equity assessment system (e.g., confidence score database 120 of FIG. 1). The database may be accessible to users of the CDP, for example, within a clinical workflow application, via a hospital network or the Internet. The users may include clinicians, researchers, product owners, data scientists, or other types of users, as described above in reference to FIG. 4. For example, the multi-dimensional confidence score and/or other health equity assessment data may be retrieved from the database and displayed in a CDP catalog, as described below in reference to FIG. 7.

Referring now to FIG. 7, an exemplary method 700 is shown for displaying health equity information generated by a health equity assessment system such as health equity assessment system 102 of FIG. 1. The health equity information may be displayed alongside CDPs in a CDP catalog, and may include a multi-dimensional confidence score, which may be generated in accordance with method 600 described above. In one embodiment, method 700 is executed by a processor of a hospital information system, such as processor 222 of healthcare information system 220 of FIG. 2. In other embodiments, method 700 may be executed by a processor of the health equity assessment system (e.g., processor 104), or a processor of a different system.

Method 700 begins at 702, where method 700 includes receiving a request to view the CDP catalog. For example, a clinician may wish to select a suitable CDP for analyzing data of a patient or cohort of patients, or a researcher may wish to review CDPs of the CDP catalog for evidence of algorithmic bias in the CDPs.

At 704, method 700 includes displaying the CDP catalog on a display screen. In various examples, the CDP catalog may be displayed within a GUI of a clinical workflow application or a hospital information system used by clinicians, researchers, and/or other users.

At 706, method 700 includes retrieving health equity assessment data associated with CDPs included in the CDP catalog from the health equity assessment system. In other words, for each CDP listed in the CDP catalog, health equity assessment data collected and generated by the health equity assessment system may be retrieved. The health equity assessment data for a CDP may include a multi-dimensional confidence score indicating, for one or more disparity factors (e.g., race, gender, age, SES, etc.), a degree of confidence of the health equity assessment system that the CDP is not algorithmically biased towards a subset of a patient population with respect to the disparity factors. The health equity assessment data for the CDP may also include other data, such as, for example, patient case or patient cohort summaries generated for patient data analyzed using the CDP.

Other health equity assessment data may also include protected characteristic labels (race and ethnicity, gender, age, disability, national origin, marital status, pregnancy or childbirth, veteran status, sexual orientation, and genetic information), and data from external data sources such as population demographic data (e.g., census data, health surveys, socioeconomic indicators); disease prevalence and outcomes data (e.g., rates and severity of diseases across different populations); and/or healthcare disparities studies (e.g., existing research on bias in healthcare access and outcomes).

At 708, method 700 includes displaying the retrieved health equity assessment data corresponding to CDPs of the CDP catalog alongside the CDPs, so that the health equity assessment data may be reviewed by a user of the CDP catalog. In some examples, a first portion of the health equity assessment data may be displayed at a "top level" of the CDP catalog, such as next to a name, identifier, or icon representing a CDP (e.g., inline), and a second portion of the health equity assessment data may be referenced via a link or control element that may display a separate page or data panel including information of the second portion. For example, the first portion of the health equity assessment data may include an icon, image, or text that summarizes the health equity information available for the CDP, and more comprehensive or substantive health equity information may be viewed when the icon, image, or text is selected. For example, the icon, image, or text may include a summary of the multi-dimensional confidence score associated with the CDP. In this way, the user of the CDP catalog may see a preview of the health equity assessment data when reviewing the CDPs, and may access more detailed data on demand.

In particular, it should be noted that the more detailed data may be advantageously displayed while the health equity assessment system is in an unlaunched state. In other words, the generation of the health equity assessment data may occur at a first time, using resources of the health equity assessment system. The health equity assessment data may be retrieved from the health equity assessment system at a second time, when the CDP catalog is displayed, using resources primarily of a relevant healthcare information system. The detailed health equity data (e.g., the second portion) may be displayed at a third time, when a user of the CDP catalog desires to see additional data. For example, the user may view a multi-dimensional confidence score displayed next to a relevant CDP, and the user may select the multi-dimensional confidence score to view specific patient data related to the multi-dimensional confidence score, such as a patient case summary indicating a specific health outcome of a patient, an output of the CDP that influenced the specific health outcome, and health disparity factors that may apply to the patient. Thus, the display of the specific data related to the multi-dimensional confidence score may not depend on the health equity assessment system being operable or accessible to the user. More specifically, the specific patient data related to the multi-dimensional confidence score may be displayed without having to request and retrieve the data from the health equity assessment system. As a result, the more detailed, second portion of health equity assessment data may be displayed in the GUI in a rapid and responsive fashion, which may encourage the user to view the health equity assessment information in detail and consider the impact of potential algorithmic bias when selecting a CDP to perform an analysis.

FIG. 11 shows an exemplary simplified CDP catalog 1100, which may be a non-limiting example of CDP catalog 226 of FIG. 2 and/or CDP catalog 404 of FIG. 4. In one embodiment, CDP catalog 1100 is accessed via a healthcare information system network and displayed within a GUI of an online resource or clinical software application, such as a clinical workflow application used by a clinician, as described above in reference to method 700 of FIG. 7. The clinician may use CDP catalog 1100 to search for a CDP to apply to patient data, for example, to aid the clinician in diagnosing a condition of a patient, determining treatment options of the patient, identifying patterns in the patient data, generating an alert if changes in the patient data are detected, or a different clinical task. In some examples, the clinician may apply the CDP to patient data of various patients (e.g., patient cohort 202). Additionally, CDP catalog 1100 may be used by other types of users, such as the clinical researchers, CDP owners, and data scientists described in reference to FIG. 4.

CDP catalog 1100 includes a plurality of CDPs, which for example, may have been licensed from a data product marketplace (e.g., CDP marketplace 224) that a relevant clinical application integrates with. The plurality of CDPs includes a first CDP 1102 titled "Silent Hypoxemia"; a second CDP 1104 titled "Ventilator Weaning Prediction Model"; and a third CDP 1106 titled "Shock". Each of CDPs 1102, 1104, and 1106 may perform a different type of analysis of patient data. For example, first CDP 1102 may be a smart alert that may be triggered in the event of silent hypoxemia, similar to the respiratory depression CDP described above in reference to FIG. 9. Second CDP 1104 may be a predictive model that returns a percentage confidence score indicating a degree to which a patient is ready to be weaned off of a ventilator. Third CDP 1106 may be a smart alert that may be triggered in the event of shock, and so on. The plurality of CDPs may be displayed in rows, where each row corresponds to a CDP included in CDP catalog 1100. Each corresponding row of a CDP may include various information about the CDP and/or control elements that may be selectable to display additional information or may be displayed in a different manner.

In particular, CDP catalog 1100 may include, for one or more CDPs included in CDP catalog 1100, a control element that provides an assessment of a health equity of the one or more CDPs performed by a health equity assessment system, such as health equity assessment system 102 of FIG. 1. For example, first CDP 1102 includes a first health equity assessment control element 1103, which may provide an indication of a confidence of the health equity assessment system that an algorithmic bias of first CDP 1102 is below a threshold level. The threshold level may be a level below which an output of first CDP 1102 may not be considered accurate for some patients, types of patients, or groups of patients. In other words, first CDP 1102 may be developed or trained based on patient data from a first narrow patient population, where the first narrow patient population may not be representative of a wider patient population. Similarly, second CDP 1104 includes a second health equity assessment control element 1105, which may provide an indication of a confidence of the health equity assessment system that an algorithmic bias of second CDP 1104 may be at or above the threshold level, and third CDP 1106 includes a third health equity assessment control element 1107, which may provide an indication of a confidence of the health equity assessment system that an algorithmic bias of third CDP 1106 may be above the threshold level.

Specifically, first health equity assessment control element 1103 includes a "thumbs up" icon 1130, which may be a visual indication that the confidence of the health equity assessment system that the algorithmic bias of first CDP 1102 is below the threshold level. First health equity assessment control element 1103 may also include a first confidence score (e.g., 95%) 1132 generated by the health equity assessment system. For example, the first confidence score may be generated by following one or more steps of method 600 of FIG 6. In the depicted embodiment, first confidence score 1132 is a single value, where the single value may be summary confidence score calculated based on a multi-dimensional confidence score outputted by the health equity assessment system.

For example, the health equity assessment system may generate a multi-dimensional confidence score comprising a first confidence dimension score based on an assessment of algorithmic bias with respect to a race of patients analyzed using first CDP 1102; a second confidence dimension score based on an assessment of algorithmic bias with respect to a gender of patients analyzed using first CDP 1102; and a third confidence dimension score based on an assessment of algorithmic bias with respect to an SES of patients analyzed using first CDP 1102. In one embodiment, first confidence score 1132 may be an average of the first confidence dimension score, the second confidence dimension score, and the third confidence dimension score. In another embodiment, first confidence score 1132 may be calculated based on the first confidence dimension score, the second confidence dimension score, and the third confidence dimension score in a different manner. For example, first confidence score 1132 may be a lowest confidence score of the first confidence dimension score, the second confidence dimension score, and the third confidence dimension score. In yet other embodiments, a plurality of confidence dimension scores of a multi-dimensional confidence score may be displayed within first health equity assessment control element 1103.

Thus, a user of CDP catalog 1100 may see at a glance a preview of health equity assessment data of first CDP 1102. Thumbs up icon 1130 may indicate that overall, algorithmic bias of CDP 1102 may be low (e.g., below the threshold), and confidence score 1132 may provide a numeric confirmation of very high confidence that CDP 1102 does not suffer from algorithmic bias.

Similar icons and confidence scores are shown for second CDP 1104 and third CDP 1106. However, second CDP 1104 indicates a confidence score 1135 of 60%, which may be around the threshold level at which an output of first CDP 1102 may not be considered accurate for some patients, types of patients, or groups of patients. Consequently, a thumbs-sideways icon 1134 is displayed. Third CDP 1106 indicates an even lower confidence score 1137 of 25%, which may be below the threshold level, whereby a thumbs-down icon 1136 is displayed.

However, an ability of a confidence score or health equity assessment to accurately reflect a probability of algorithmic bias in a CDP may depend on an amount of patient data considered in a health equity assessment performed by the health equity assessment system. For example, a first health equity assessment based on patient data from hundreds of patients may be more accurate than a second health equity assessment based on patient data from one or a small number of patients. Alternatively, a CDP may have a high confidence score based on data from hundreds of patients, but the hundreds of patients may be drawn from a narrow patient population. For example, the high confidence score may be based on a large amount of patient data of Caucasian patients, but a very small amount of patient data of African-American patients, where an analysis generated by the CDP for an African-American patient may not be as accurate as an analysis generated by the CDP for a Caucasian patient. As a result, determining whether algorithmic bias of a CDP may be significant enough to disqualify the CDP from being used with a given patient, or whether parameters of the CDP could be changed to reduce the algorithmic bias, may include reviewing the specific patient data used by the health equity assessment system to generate the assessment and/or confidence score.

To allow a user of CDP catalog 1100 to review the specific patient data, a case count element 1110 may be included for one or more CDPs, that indicates a number of patients or patient cases considered by the health equity assessment system in the generation of the health equity assessment and/or confidence score(s). For example, in the depicted embodiment, second CDP 1104 includes a second health equity assessment control element 1105 including a second confidence score (e.g., 60%) 1135 generated by the health equity assessment system, where a case count element 1110 indicates that the confidence score of 60% displayed for second CDP 1104 is based on four patient cases. The user may select the case count element 1110 or second health equity assessment control element 1105 to view the four patient cases.

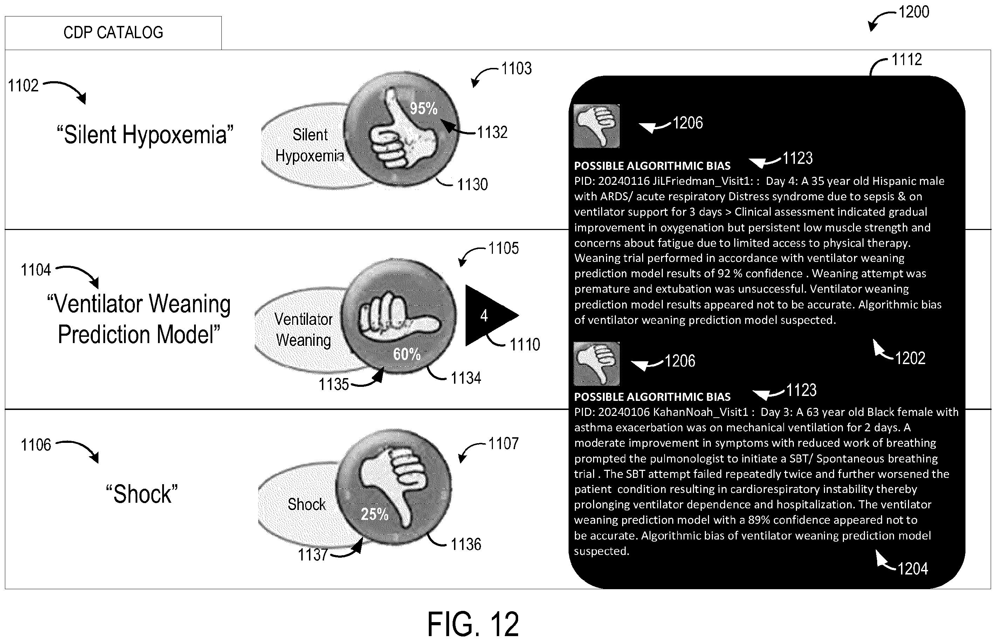

When the user selects the case count element 1110, a patient case summary panel 1112 may be displayed (e.g., as a pop-up window, or superimposed over CDP catalog 1100, or in an expanded portion of a row of CDP catalog 1100, etc.). Patient case summary panel 1112 may include various patient case summaries, which may be displayed in a scrollable display, or a series of pages, or in a series of nested pages that can be drilled down. In the depicted example, patient case summary panel 1112 may include a first patient case summary 1120 of the four patient cases, and a second patient case summary 1122 of the four patient cases, with the remaining two patient case summaries on a subsequent page shown in FIG. 12.

In various embodiments, first patient case summary 1120 and second patient case summary 1122 may be generated by a clinical context summarizer of the health equity assessment system, such as clinical context summarizer 116 of FIG. 1. The clinical context summarizer may generate first patient case summary 1120 and second patient case summary 1122 (and other patient case summaries) using an LLM, as described above.

The user may first review the four patient case summaries to determine a basis for potential algorithmic bias indicated in the second confidence score 1135. The user may see that first patient case summary 1120 relates to a patient who is a 58-year-old Caucasian man, where an algorithmic bias was not detected. The user may see that second patient case summary 1122 relates to a patient who is a 42-year-old Caucasian woman, where an algorithmic bias was not detected. A thumbs-up icon 1121 may be displayed adjacent to the first patient case summary 1120 and the second patient case summary as a result of the algorithmic bias not being detected. Additionally or alternatively, an algorithmic summary statement 1123 may be displayed for one or more patient case summaries.

The user may view the additional patient cases by selecting a "MORE" link 1124, or similar control element, which may cause one or more new patient cases to be displayed in a subsequent page of patient case summary panel 1112. In other examples, the user may scroll down patient case summary panel 1112 by manipulating scroll bars as known in the art, or additional case summaries may be displayed in a different manner.

FIG. 12 shows a first scrolled view 1200 of simplified CDP catalog 1100, where a third patient case summary 1202 of the four patient cases is displayed on patient case summary panel 1112. The user may review third patient case summary 1202 to determine a basis for potential algorithmic bias indicated in the second confidence score 1135. The user may see that a possible algorithmic bias was detected, which may be indicated by a relevant algorithmic summary statement 1123 and/or a thumbs-down icon 1206. The user may see that third patient case summary 1202 refers to a 35-year-old Hispanic man, where the ventilator weaning model predicted that a weaning trial should be performed with a 92% confidence, yet despite the result, the extubation was unsuccessful. As a result, the clinician concluded that algorithmic bias may be a cause of the incorrect prediction, where a population used to train the ventilator weaning model may not have included significant numbers of non-white subjects during training.

The user may similarly review a fourth patient case summary 1204 to determine a basis for potential algorithmic bias indicated in the second confidence score 1135. The user may see that a possible algorithmic bias was detected, which may be indicated by a relevant algorithmic summary statement 1123 and/or a thumbs-down icon 1206. The user may see that fourth patient case summary 1204 refers to a 63-year-old African-American woman, where the ventilator weaning model predicted that a weaning trial should be performed with a 89% confidence, yet despite the result, a spontaneous breathing trial failed repeatedly, resulting in prolonged ventilator dependence and hospitalization. As a result, the clinician concluded that algorithmic bias may be a cause of the incorrect prediction, where a population used to train the ventilator weaning model may not have included significant numbers of non-white subjects during training.

In this way, the user may determine the reasons for the relatively low confidence score of 60% for second confidence score 1135: of four cases, the ventilator weaning prediction model made accurate predictions for two Caucasian patients, and made inaccurate predictions for two non-Caucasian patients. As a result, the user may take one of several actions. The user may be a clinician, and may decide not to use second CDP 1104 on a patient of the user, due to a potential defect of the model (e.g., the algorithmic bias). Alternatively, the clinician may decide not to use second CDP 1104 on patients of color, but to use second CDP 1104 on Caucasian patients. In a third case, the clinician may decide to use second CDP 1104 on a patient who is non-Caucasian, but the clinician may be cautious about interpreting a result of second CDP 1104, to generate more data from which a more accurate assessment of second CDP 1104 may be made, as due to the low number of available cases, sufficient information may not be available to infer the algorithmic bias with confidence.

In some embodiments, other health equity assessment data may be displayed or provided by selecting the case count element 1110 or second health equity assessment control element 1105. For example, the user may select the case count element 1110 to view text summaries of patient cases analyzed to generate confidence score 1135, and the user may select second health equity assessment control element 1105 to view other health equity assessment data associated with confidence score 1135. For example, the patient data used to generate the health equity assessment of second CDP 1104 may include too many patient cases to display efficiently in patient case summary panel 1112. In such cases, a textual summary of the patient data may be displayed in patient case summary panel 1112 (e.g., instead of individual patient cases), and additional information about how confidence score 1135 was generated may be displayed in an alternative or additional display panel. The additional information (e.g., the second portion described above in reference to method 700) may include, for example, statistics and/or demographic data showing how a total patient population analyzed by the health equity assessment system may be broken down into sub-groups representing different disparity factors. The additional information may include multi-dimensional confidence scores that show different confidence dimensions corresponding to the different disparity factors. The additional information may include visual elements such as graphs, charts, tables, etc. that may summarize the patient data. Additionally, the additional information and/or the visual elements may also be selectable by the user, such that the user may navigate health equity assessment data (including patient data) in a "drill-down" fashion, where data of increasing granularity is displayed based on user selections.

Additionally, it should be appreciated that the health equity assessment scores, ratings, patient case summaries, and additional related data may be retrieved from the health equity assessment system at a time when CDP catalog 1100 is generated or displayed. When CDP catalog 1100 is displayed, a preview or summary of the health equity assessment data may be displayed at a top level, meaning, next to CDPs displayed in CDP catalog 1100. The user may then view more detailed health equity assessment data by selecting the preview, and the more detailed health equity assessment data may be displayed to the user without communicating with the health equity assessment system. For example, the health equity assessment system may be in an unlaunched state and inaccessible to the user after the generation of CDP catalog 1100. In this way, an efficiency of a functioning of a computing device displaying CDP catalog 1100 may be increased. That is, in an alternate scenario where CDP catalog 1100 was generated with the previewed, summarized health equity assessment data and not with the more detailed health equity assessment data, to view the more detailed health equity assessment data, the user would select the previewed, summarized health equity assessment data (e.g., the confidence score), and the more detailed health equity assessment data would be retrieved on demand from the health equity assessment system. As a result of retrieving the more detailed health equity assessment data on demand, a consumption of processing power of the computing device would be increased while the user is assessing algorithmic bias of the CDP, which may increase an amount of time taken by the user to assess the algorithmic bias and discourage the user from requesting additional data.

In other words, the methods disclosed herein for generating and displaying the health equity assessment information at a level of detail desired by the user improve the capabilities of both the health equity assessment system and the healthcare information system displaying the CDP catalog, by reducing an amount of processing that would otherwise be performed on demand when compiling the health equity assessment data from hospital databases or retrieving various elements of the health equity assessment data from the health equity assessment system. The health equity assessment data is generated in a first step by the health equity assessment system using one or more disparity assessment models. The first portion of the health equity assessment data, which may be a preview summarizing the health equity assessment data, is associated with the CDPs in a second step, when the CDP catalog is displayed or populated with CDPs. The more detailed, second portion of the health equity assessment data corresponding to the preview may be displayed to the user, upon selection of a CDP, at a third step, which may be at a later time, during which the health equity assessment system may not be accessible or even running. Thus, if the user wishes to view the more detailed, second portion of the health equity assessment data corresponding to the preview, the detailed health equity assessment data can be generated quickly via controls presented in the GUI, with minimal processing. In this way, the dynamic display of the health equity assessment data improves the way the health equity assessment system and the healthcare information system store and retrieve data in memory to reduce resource consumption. A specific manner of displaying health equity assessment data to the user based on a limited set of data is described, such that the user is not burdened by time-consuming, iterative calculations, or by navigating through pages of health equity assessment data amassed for different patients or products. Because the user is not forced to scroll down or navigate through various layers of data to view the health equity assessment data, a rapid and efficient process for reviewing a potential algorithmic bias of a CDP is enabled. As a result, CDPs with a lower probability of algorithmic bias may be selected, and in shorter time frames, than would be permitted by prior art systems and GUIs. Thus, the disclosed invention improves the efficiency of the health equity assessment system and healthcare information systems that rely on the health equity assessment system.

The technical effect of collecting specific disparity factor data applicable to assessing a CDP for algorithmic bias, and converting the data to a highly standardized format, is that memory and processing resources of healthcare information systems used to analyze the data for algorithmic bias may be reduced, in comparison with current methods that rely on sifting through and cross-referencing data already stored in different databases of a healthcare system.

The disclosure also provides support for a health equity assessment system, comprising: a processor and a non-transitory memory storing instructions that when executed, cause the processor to: summarize a clinical context of an analysis performed by a clinical data product (CDP) on patient data, perform a health equity assessment of the CDP based on the summarized clinical context, using one or more disparity assessment models trained to assess an algorithmic bias of the CDP towards a specific patient population, calculate a confidence score for the CDP based on the health equity assessment, the confidence score indicating a degree of confidence that an analysis of a patient using the CDP does not suffer from algorithmic bias, and store the confidence score in a database. In a first example of the system, the summarized clinical context of the analysis performed by the CDP on the patient data includes demographic data with respect to a plurality of disparity factors of a patient population for which the algorithmic bias may be detected, the disparity factors including at least race, gender, age, disability, social determinants of health (SDOH), and socio-economic status (SES). In a second example of the system, optionally including the first example, the confidence score is a multi-dimensional confidence score comprising a plurality of confidence scores for a respective plurality of disparity factors. In a third example of the system, optionally including one or both of the first and second examples, the clinical context of the analysis performed by the CDP on patient data is summarized by a large language model (LLM) that generates a natural language text summary of the patient data. In a fourth example of the system, optionally including one or more or each of the first through third examples, the clinical context of the analysis performed by the CDP on patient data is summarized into a structured text file including labeled data in a standardized format, the labeled data used as input data for a disparity assessment model. In a fifth example of the system, optionally including one or more or each of the first through fourth examples, further instructions are stored in the non-transitory memory that when executed, cause the processor to perform the health equity assessment of the CDP in response to receiving clinical feedback of an output of the CDP by a clinician of a healthcare system during the analysis performed on the patient data by the clinician using the CDP, the received clinical feedback including demographic data with respect to the plurality of disparity factors. In a sixth example of the system, optionally including one or more or each of the first through fifth examples, the clinical feedback includes a health equity rating of the CDP generated by the clinician via a CDP rating tool micro-application of the health equity assessment system opened within a software application used by the clinician to perform the analysis of the patient data using the CDP. In a seventh example of the system, optionally including one or more or each of the first through sixth examples, the CDP rating tool micro-application displays: a first control element that when selected, sends a positive rating of the output of the CDP to the health equity assessment system, a second control element that when selected, sends a negative rating of the output of the CDP to the health equity assessment system, a third control element that when selected, sends an indication that sufficient information is not available to rate the output of the CDP to the health equity assessment system, and a fourth control element that allows a user of the CDP rating tool micro-application to send text data regarding the output of the CDP to the health equity assessment system. In a eighth example of the system, optionally including one or more or each of the first through seventh examples, the system further comprises: a model drift detector configured to monitor health equity assessments and trends in confidence ratings of the CDP to determine whether a disparity assessment model of the one or more disparity assessment models is becoming progressively less accurate due to algorithmic bias. In a ninth example of the system, optionally including one or more or each of the first through eighth examples, the model drift detector is further configured to measure a performance of the CDP against a baseline performance of the CDP in accordance with a plurality of key metrics, with respect to at least one of: changes in a distribution of input data used by the CDP over time, changes in a relative importance of different features of the CDP over time, and changes in predictions or outputs of the CDP over time. In a tenth example of the system, optionally including one or more or each of the first through ninth examples, the key metrics include at least one of: an accuracy, precision, or recall of a disparity assessment model, an output of a drift detection algorithm, a Kolmogorov-Smirnov (KS) Test, a Earth Mover's Distance (EMD), a Jensen-Shannon Divergence (JSD), a Chi-squared Test, a Wilcoxon signed-rank test, a Population Stability Index (PSI), and an area under a receiver operating characteristic curve (AUC-ROC). In a eleventh example of the system, optionally including one or more or each of the first through tenth examples, the CDP is selected by a clinician from a CDP catalog that displays a list of CDPs available to the clinician, and for each CDP of the list of CDPs, a preview of health equity assessment data available for the CDP.

The disclosure also provides support for a machine-implemented method for a health equity assessment system of a healthcare system, the method comprising: receiving a rating of a clinical data product (CDP) used by a clinician to analyze patient data, summarizing a clinical context of an analysis performed by the CDP on the patient data, performing a health equity assessment of the CDP, the health equity assessment evaluating the summarized clinical context using one or more disparity assessment models to assess an algorithmic bias of the CDP, calculating a confidence score for the CDP, based on the assessed algorithmic bias, and storing the confidence score in a database. In a first example of the method, summarizing the clinical context of the analysis performed by the CDP on the patient data further comprises summarizing the clinical context into a text summary including demographic data with respect to a plurality of disparity factors of a patient population for which the algorithmic bias may be detected, the disparity factors including at least race, gender, age, disability, social determinants of health (SDOH), and socio-economic status (SES). In a second example of the method, optionally including the first example, the confidence score is a multi-dimensional confidence score comprising a plurality of confidence scores for a respective plurality of disparity factors. In a third example of the method, optionally including one or both of the first and second examples, summarizing the clinical context into the text summary further comprises using a large language model (LLM) to generate a natural language text summary of the patient data. In a fourth example of the method, optionally including one or more or each of the first through third examples, the natural language text summary is included in a listing of the CDP in a CDP catalog displayed to clinicians. In a fifth example of the method, optionally including one or more or each of the first through fourth examples,: the listing of the CDP in the CDP catalog is configured to display a preview of the natural language text summary, the preview including at least a number of patient cases included in the natural language text summary, and the natural language text summary is displayed in display panel of a graphical user interface (GUI) of the CDP catalog that can be reached directly by selecting the preview while the health equity assessment system is in an unlaunched state.

The disclosure also provides support for a clinical data product (CDP) rating tool micro-application, comprising a rating tool launched within a software application used by a clinician to perform an analysis of patient data using the CDP, the rating tool configured to: receive clinical feedback from the clinician regarding an algorithmic bias of the CDP, and send the clinical feedback to a health equity assessment system configured to assess the algorithmic bias of the CDP. In a first example of the system, the rating tool includes: a first control element that when selected, sends a positive rating of an output of the CDP to the health equity assessment system, a second control element that when selected, sends a negative rating of the output of the CDP to the health equity assessment system, a third control element that when selected, sends an indication that sufficient information is not available to rate the output of the CDP to the health equity assessment system, and a fourth control element that allows a user of the rating tool micro-application to send text data regarding the output of the CDP to the health equity assessment system.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative and should not be construed to be limiting in any manner.

## Claims

1. A health equity assessment system (102, 214), comprising:
a processor (104) and a non-transitory memory (106) storing instructions that when executed, cause the processor (104) to:
summarize a clinical context of an analysis performed by a clinical data product (CDP (904, 1102, 206, 175, 302, 402)) on patient data (902, 204);
perform a health equity assessment (408) of the CDP (904, 1102, 206, 175, 302, 402) based on the summarized clinical context, using one or more disparity assessment models (110) trained to assess an algorithmic bias of the CDP (904, 1102, 206, 175, 302, 402) towards a specific patient population;
calculate a confidence score (1132, 1135) for the CDP (904, 1102, 206, 175, 302, 402) based on the health equity assessment (408), the confidence score (1132, 1135) indicating a degree of confidence that an analysis of a patient using the CDP (904, 1102, 206, 175, 302, 402) does not suffer from algorithmic bias; and
store the confidence score (1132, 1135) in a database.

2. The health equity assessment system (102, 214) of claim 1, wherein the summarized clinical context of the analysis performed by the CDP (904, 1102, 206, 175, 302, 402) on the patient data (902, 204) includes demographic data with respect to a plurality of disparity factors of a patient population for which the algorithmic bias may be detected, the disparity factors including at least race, gender, age, disability, social determinants of health (SDOH), and socio-economic status (SES).

3. The health equity assessment system (102, 214) of claim 2, wherein the confidence score (1132, 1135) is a multi-dimensional confidence score (1132, 1135) comprising a plurality of confidence scores for a respective plurality of disparity factors.

4. The health equity assessment system (102, 214) of claim 1, wherein the clinical context of the analysis performed by the CDP (904, 1102, 206, 175, 302, 402) on patient data (902, 204) is summarized by a large language model (LLM) that generates a natural language text summary of the patient data (902, 204).

5. The health equity assessment system (102, 214) of claim 1, wherein the clinical context of the analysis performed by the CDP (904, 1102, 206, 175, 302, 402) on patient data (902, 204) is summarized into a structured text file including labeled data in a standardized format, the labeled data used as input data for a disparity assessment model.

6. The health equity assessment system (102, 214) of claim 2, wherein further instructions are stored in the non-transitory memory (106) that when executed, cause the processor (104) to perform the health equity assessment (408) of the CDP (904, 1102, 206, 175, 302, 402) in response to receiving clinical feedback of an output of the CDP (904, 1102, 206, 175, 302, 402) by a clinician (416, 207) of a healthcare system during the analysis performed on the patient data (902, 204) by the clinician (416, 207) using the CDP (904, 1102, 206, 175, 302, 402), the received clinical feedback including demographic data with respect to the plurality of disparity factors.

7. The health equity assessment system (102, 214) of claim 6, wherein the clinical feedback includes a health equity rating of the CDP (904, 1102, 206, 175, 302, 402) generated by the clinician (416, 207) via a CDP rating tool micro-application of the health equity assessment system (102, 214) opened within a software application used by the clinician (416, 207) to perform the analysis of the patient data (902, 204) using the CDP (904, 1102, 206, 175, 302, 402).

8. The health equity assessment system (102, 214) of claim 7, wherein the CDP rating tool micro-application displays:
a first control element (1002) that when selected, sends a positive rating of the output of the CDP (904, 1102, 206, 175, 302, 402) to the health equity assessment system (102, 214);
a second control element (1004) that when selected, sends a negative rating of the output of the CDP (904, 1102, 206, 175, 302, 402) to the health equity assessment system (102, 214);
a third control element (1006) that when selected, sends an indication that sufficient information is not available to rate the output of the CDP (904, 1102, 206, 175, 302, 402) to the health equity assessment system (102, 214); and
a fourth control element (1008) that allows a user of the CDP rating tool micro-application to send text data regarding the output of the CDP (904, 1102, 206, 175, 302, 402) to the health equity assessment system (102, 214).

9. The health equity assessment system (102, 214) of claim 6, further comprising a model drift detector (114) configured to monitor health equity assessments (408) and trends in confidence ratings of the CDP (904, 1102, 206, 175, 302, 402) to determine whether a disparity assessment model of the one or more disparity assessment models (110) is becoming progressively less accurate due to algorithmic bias.

10. The health equity assessment system (102, 214) of claim 9, wherein the model drift detector (114) is further configured to measure a performance of the CDP (904, 1102, 206, 175, 302, 402) against a baseline performance of the CDP (904, 1102, 206, 175, 302, 402) in accordance with a plurality of key metrics, with respect to at least one of:
changes in a distribution of input data used by the CDP (904, 1102, 206, 175, 302, 402) over time;
changes in a relative importance of different features of the CDP (904, 1102, 206, 175, 302, 402) over time; and
changes in predictions or outputs of the CDP (904, 1102, 206, 175, 302, 402) overtime.

11. The health equity assessment system (102, 214) of claim 10, wherein the key metrics include at least one of:
an accuracy, precision, or recall of a disparity assessment model;
an output of a drift detection algorithm;
a Kolmogorov-Smirnov (KS) Test;
an Earth Mover's Distance (EMD);
a Jensen-Shannon Divergence (JSD);
a Chi-squared Test;
a Wilcoxon signed-rank test;
a Population Stability Index (PSI); and
an area under a receiver operating characteristic curve (AUC-ROC).

12. The health equity assessment system (102, 214) of claim 1, wherein the CDP (904, 1102, 206, 175, 302, 402) is selected by a clinician (416, 207) from a CDP catalog (226, 176, 404, 1100) that displays a list of CDPs (1102, 206, 175, 402) available to the clinician (416, 207), and for each CDP (904, 1102, 206, 175, 302, 402) of the list of CDPs, a preview of health equity assessment data available for the CDP (904, 1102, 206, 175, 302, 402).

13. A machine-implemented method (600) for a health equity assessment system of a healthcare system, the method comprising:
receiving a rating of a clinical data product (CDP) used by a clinician to analyze patient data (602);
summarizing a clinical context of an analysis performed by the CDP on the patient data (604);
performing a health equity assessment of the CDP (608), the health equity assessment evaluating the summarized clinical context using one or more disparity assessment models to assess an algorithmic bias of the CDP;
calculating a confidence score for the CDP (614), based on the assessed algorithmic bias; and
storing the confidence score in a database (616).

14. The machine-implemented method (600) of claim 13, wherein summarizing the clinical context of the analysis performed by the CDP on the patient data further comprises summarizing the clinical context into a text summary including demographic data with respect to a plurality of disparity factors of a patient population for which the algorithmic bias may be detected, the disparity factors including at least race, gender, age, disability, social determinants of health (SDOH), and socio-economic status (SES).

15. The machine-implemented method of claim 14, wherein the confidence score is a multi-dimensional confidence score comprising a plurality of confidence scores for a respective plurality of disparity factors.
